# EUROPEAN PATENT APPLICATION

(11) **EP 4 841 644 A1**
(43) Date of publication of application: **18.11.2026**
(21) Application number: 25305680.8
(22) Date of filing: 13.05.2025
(51) Int. Cl.: G16H 50/20, G16H 50/50, G16H 50/70

(54) **PREDICTION OF A HEMODYNAMIC VASCULAR BEHAVIOR OF A PATIENT WITH UNCERTAINTY QUANTIFICATION**

(71) Applicant: Dassault Systèmes, 78140 Vélizy-Villacoublay (FR)
(72) Inventor: BENMAHDI, Meryem, 78140 Velizy-Villacoublay (FR)
(74) Representative: Bandpay & Greuter

(57) **Abstract**

The disclosure notably relates to a computer-implemented method for predicting a hemodynamic vascular behavior of a plurality of arteries of a patient with uncertainty quantification. The method comprises obtaining one or more pre-trained machine-learning functions. The method further comprises obtaining measurements of the hemodynamical vascular behavior of the patient with a medical measuring device and generating, from the obtained measurements, a plurality of noisy measurements of the hemodynamic vascular behavior of the patient, the generating comprising applying the obtained pre-trained machine-learning functions. The method further comprises applying the obtained pre-trained machine-learning functions to the plurality of noisy measurements, thereby obtaining a plurality of patient-dependent instances of the predetermined hemodynamic signals. The method thereby constitutes an improved solution to predicting a hemodynamic vascular behavior of a plurality of arteries of a patient, in particular with uncertainty quantification.

## Description

### TECHNICAL FIELD

The disclosure relates to the field of computer programs and systems, and more specifically to a method, system and program for predicting a hemodynamic vascular behavior of a plurality of arteries of a patient with uncertainty quantification.

### BACKGROUND

A number of systems and programs are offered on the market of healthcare industry to support healthcare professionals in the diagnosis, interventional planning, and/or follow-up of patients. Such offered systems and programs - which may be also known as healthcare software solutions - are developed for simulation and medical training, research, diagnosis, database storage and/or equipment planning. Digital or equivalently virtual twins are among such offered systems and programs. Digital twins provide a virtual representation (i.e., the "digital twin") of patients ("physical twin"). Such digital twins are generated from patient data, and real-time updates on patient and environmental variables based on a set of physics-based simulations. Digital twins seek to match clinical measurements of the patient's anatomical and physiological features to provide a personalized and reliable model for each patient.

Personalizing a digital twin for a patient involves several measurement uncertainties that influence the reliability of the predictions and that a healthcare professional must carefully consider while evaluating predictions. For example, a medical measuring device used to obtain measurements of a hemodynamical vascular behavior of a patient may measure said behavior at a section of an artery. However, the exact position of the section of the artery may not be obtainable with the medical measuring device (e.g., without intrusive measures). As the hemodynamical vascular behavior is different in different sections of the artery (due, e.g., to boundary conditions), the uncertainty of this exact position may impact the digital model reliability.

Within this context, there is still a need for an improved prediction of a hemodynamic vascular behavior of a plurality of arteries of a patient, in particular with uncertainty quantification.

### SUMMARY

It is therefore provided a computer-implemented method for predicting a hemodynamic vascular behavior of a plurality of arteries of a patient with uncertainty quantification. The method comprises obtaining one or more pre-trained machine-learning functions.

The obtaining of one or more pre-trained machine-learning functions comprises obtaining a pre-trained parameter-signal machine-learning function. The pre-trained parameter-signal machine-learning function has been trained for outputting an instance of predetermined hemodynamic signals from an instance of predetermined parameters provided as input. Wherein the predetermined hemodynamic signals comprise one or more hemodynamic signals. Each of the hemodynamic signals characterizes a blood flow of a respective artery of the plurality of arteries of the patient. Wherein the predetermined parameters comprise one or more parameters. Each of the parameters characterizes a respective artery of the plurality of arteries of the patient. The obtaining of one or more pre-trained machine-learning functions further comprises obtaining a pre-trained parameter-noise machine-learning function. The pre-trained parameter-noise machine-learning function has been trained for outputting an instance of predetermined characterizations of hemodynamic localization noise from an instance of the predetermined parameters provided as input. Wherein the predetermined characterizations of hemodynamic localization noise comprise one or more characterizations of noise. Each characterization of noise characterizes a variability of a hemodynamic signal characterizing a blood flow of a respective artery of the plurality of arteries of the patient. The variability of the hemodynamic signal characterizing the blood flow is with respect to a location in the artery. The obtaining of one or more pre-trained machine-learning functions further comprises obtaining a pre-trained measurement-parameter machine-learning function. The pre-trained measurement-parameter machine-learning function has been trained for outputting an instance of the predetermined parameters from measurements of the hemodynamic vascular behavior provided as input. Wherein the measurements of the hemodynamic vascular behavior comprise instances of hemodynamic signals characterizing the blood flow of a predetermined subset of arteries of the plurality of arteries of the patient.

The method further comprises obtaining measurements of the hemodynamical vascular behavior of the patient with a medical measuring device. The method further comprises generating a plurality of noisy measurements of the hemodynamic vascular behavior of the patient.

The generating of a plurality of noisy measurements comprises providing as input the obtained measurements of the hemodynamic vascular behavior of the patient to the pre-trained measurement-parameter machine-learning function. Thereby the method obtains a patient-dependent instance of the predetermined parameters. The generating of a plurality of noisy measurements further comprises providing as input the obtained patient-dependent instance of the predetermined parameters to the pre-trained parameter-noise machine-learning function. Thereby the method obtains a patient-dependent instance of the predetermined characterizations of hemodynamic localization noise. The generating of a plurality of noisy measurements further comprises determining a patient-dependent noise generator from the patient-dependent instance of the predetermined characterizations of hemodynamic localization noise. The patient-dependent noise generator is able to modify the measurements of the hemodynamic vascular behavior of the patient by adding noise. The noise quantifies possible errors in the measurements of the hemodynamic vascular behavior of the patient with the medical measuring device.

The method further comprises providing as input each of the noisy measurements of the hemodynamical vascular behavior of the patient to the pre-trained measurement-parameter machine-learning function. The method thereby obtains a plurality of noisy patient-dependent instances of the predetermined parameters. The method further comprises providing as input each of the obtained plurality of noisy patient-dependent instances of the predetermined parameters to the pre-trained parameter-signal machine-learning function. The method thereby obtains a plurality of noisy patient-dependent instances of the predetermined hemodynamic signals.

The method may comprise one or more of the following:
- obtaining a noise confidence level of the measurements of the hemodynamical vascular behavior of the patient with the medical measuring device, and wherein the patient-dependent noise generator is further determined from the obtained noise confidence level;
- the pre-trained parameter-signal machine-learning function comprises a pre-trained parameter-signal neural network model for one or more of the hemodynamic signals of respective arteries of the plurality of arteries of the patient comprised in the predetermined hemodynamic signals, each of said pre-trained parameter-signal neural network models outputting instances of the hemodynamic signal of the respective artery;
- the pre-trained parameter-noise machine-learning function comprises a pre-trained parameter-noise neural network model for one or more characterization of noise characterizing a variability of a hemodynamic signal characterizing a blood flow of a respective artery of the plurality of arteries of the patient comprised in the predetermined characterizations of hemodynamic localization noise, each of said pre-trained parameter-noise neural network models outputting instances of the characterization of noise of the respective artery;
- the pre-trained measurement-parameter machine-learning function comprises a pre-trained measurement-parameter neural network model for one or more parameters characterizing a respective artery of the plurality of arteries of the patient comprised in the predetermined parameters, each of said pre-trained measurement-parameter neural network models outputting instances of the parameter characterizing the respective artery;
- the obtaining (S101) of the pre-trained parameter-signal machine-learning function comprises:
   ∘ obtaining (S151) a parameter-signal dataset comprising entries, each entry comprising an instance of the predetermined parameters and an instance of the predetermined hemodynamic signals; and
   ∘ training (S161) a machine-learning function on the parameter-signal dataset, thereby obtaining the pre-trained parameter-signal machine-learning function;
- the obtaining (S151) of the parameter-signal dataset comprises:
   ∘ obtaining numerical bounds for each parameter comprised in the predetermined parameters, each of the numerical bounds determining a physiological plausible range of the possible values of the respective parameter in a population;
   ∘ generating a list of instances of the predetermined parameters, each instance comprising an instance of each of the parameters comprised in the predetermined parameters, the instance of each of the parameters being generated randomly within the obtained numerical bounds;
   ∘ obtaining for each instance of the predetermined parameters comprised in the generated list an instance of the predetermined hemodynamic signals by using a one-dimensional blood flow modeling solver;
   ∘ normalizing each of the instances of each of the parameters comprised in the instances of the predetermined parameters comprised in the generated list and each of the instances of each of the hemodynamic signals comprised in the obtained instances of predetermined hemodynamic signals; and optionally wherein
   ∘ the one-dimensional blood flow modeling solver is given by the formula: $\left\{\begin{matrix}\frac{\partial A}{\partial t} + \frac{\partial Q}{\partial x} = 0 \\ \frac{\partial Q}{\partial t} + \frac{\partial }{\partial x} \left(α\frac{{Q}^{2}}{A}\right) + \frac{A}{ρ} \frac{\partial P}{\partial x} = - 2 \frac{μ}{ρ} \left({γ}_{v}+2\right) \frac{Q}{A}\end{matrix}\right.$ where *A* is the cross-sectional area, *Q* is the blood flow-rate, *t* is the time coordinate, *x* is the position within the artery, *P* is the blood pressure, *α* is a parameter that reflects the radial profile of the longitudinal velocity, *ρ* is the mass density, *µ* is the dynamic viscosity, and ${γ}_{ν} = \frac{\left(α-2\right)}{1 - α}$;
- the obtaining (S102) of the pre-trained parameter-noise machine-learning function comprises:
   ∘ obtaining (S152) a parameter-noise dataset, the parameter-noise dataset comprising entries, each entry comprising an instance of the predetermined parameters and an instance of the predetermined characterizations of hemodynamic localization noise; and
   ∘ training (S162) a machine-learning function on the parameter-noise dataset, thereby obtaining the pre-trained parameter-noise machine-learning function;
- the obtaining (S152) of the parameter-noise dataset comprises for each given entry of the obtained parameter-signal dataset:
   ∘ determining a covariance matrix of each of the instances of the hemodynamic signals associated to the given entry with respect to the localization; and optionally wherein
   ∘ the covariance matrix *C^{loc}*(*t, t'*|*p*) is given by the formula: $\begin{matrix}{C}^{loc} \left(t,t′\left|p\right.\right) = {E}_{x} \left[\left(S\left(x,t\left|p\right.\right)-{E}_{x}\left[S\left(x,t\left|p\right.\right)\right]\right)\left(S\left(x,t′\left|p\right.\right)-{E}_{x}\left[S\left(x,t′\left|p\right.\right)\right]\right)\right] \\ = {E}_{x} \left[S\left(x,t\left|p\right.\right)S\left(x,t′\left|p\right.\right)\right] - {E}_{x} \left[S\left(x,t\left|p\right.\right)\right] {E}_{x} \left[S\left(x,t′\left|p\right.\right)\right]\end{matrix}$ where *S* is the instance of the hemodynamic signal characterizing the blood flow, *t* and *t'* are time coordinates, x is the position within the artery, and *p* is the instance of the predetermined parameters comprised in the entry;
- the obtaining (S103) of the pre-trained measurement-parameter machine-learning function comprises:
   ∘ obtaining (S153) a measurement-parameter dataset, the measurement-parameter dataset comprising entries, each entry comprising measurements of the hemodynamic vascular behavior and an instance of the predetermined parameters; and
   ∘ training (S163) a machine-learning function on the measurement-parameter dataset, thereby obtaining the pre-trained measurement-parameter machine-learning function;
- the obtaining (S153) of the measurement-parameter dataset comprises for each given entry of the obtained parameter-signal dataset:
   ∘ obtaining measurements of the hemodynamic vascular behavior from the instances of the hemodynamic signals characterizing the blood flow of the predetermined subset of arteries comprised in the given entry;
   ∘ obtaining noisy measurements of the hemodynamic vascular behavior by adding to the obtained measurements of the hemodynamic vascular a noise;
   ∘ solving an inverse problem using Bayes method, thereby obtaining an instance of the predetermined parameters that are associated with the noisy measurements of the hemodynamic vascular behavior; and optionally wherein
   ∘ solving the inverse problem comprises solving a non-linear optimization problem by minimizing the negative log-posterior using the formula ${p}^{MAP} = {argmin}_{p ∈ {ε}_{p}} \left(-{\sum }_{i = 1}^{{N}_{O}} \left(\begin{matrix}- \frac{{N}_{T}}{2} log \left(2π\right) - \frac{1}{2} log \left(\left|Σ\right|\right) \\ - \frac{1}{2} {\left({O}_{i}-{M}_{i}^{NN}\left(p\right)\right)}^{T} {Σ}^{- 1} \left({O}_{i}-{M}_{i}^{NN}\left(p\right)\right)\end{matrix}\right) - log \left({π}_{prior}\left(p\right)\right)\right)$ where:
      ▪ *p^{MAP}* is the instance of the predetermined parameters that are associated with the noisy measurements,
      ▪ *εₚ* is a space of all the possible instances of the predetermined parameters,
      ▪ *p* is an instance of the predetermined parameters,
      ▪ *N_{O}* is a number of hemodynamic signals characterizing the blood flow of a predetermined subset of arteries of the plurality of arteries of the patient,
      ▪ *N_{T}* is a number of time steps of each of the hemodynamic signals,
      ▪ *Σ* is a covariance matrix characterizing the noise,
      ▪ *Oᵢ* is an instance of a *i*-th hemodynamic signal of the noisy measurements of the hemodynamic vascular behavior,
      ▪ ${M}_{i}^{NN} \left(p\right)$ is an instance of the *i*-th hemodynamic signal of the measurements of the hemodynamic vascular behavior obtained from the instance *p* of the predetermined parameters, and
      ▪ *πₚᵣᵢₒᵣ*(*p*) is the priority density of the parameters;
- each characterization of noise comprised in the predetermined characterizations of hemodynamic localization noise is a covariance matrix, and the patient-dependent noise generator comprises one or more multivariate normal distributions, each of the multivariate normal distribution determined by an instance of a covariance matrix comprised in the patient-dependent instance of the predetermined characterizations of hemodynamic localization noise;
- the predetermined hemodynamic signals comprise one or more selected among blood pressure, flow rate, cross-sectional area, velocity of each artery of the plurality of arteries of the patient; and/or
- the predetermined parameters comprise one or more selected among an anatomical, a physiological, a physical, a model parameters of each artery of the plurality of arteries of the patient, and optionally wherein the predetermined parameters comprise one or more selected among a Young's modulus, a vessel length, a vessel radius, 0D Windkessel model parameters of each artery of the plurality of arteries of the patient.

It is further provided a computer program comprising instructions for performing the method.

It is further provided a computer readable storage medium having recorded thereon the computer program.

It is further provided a system comprising a processor coupled to a memory and a graphical user interface, the memory having recorded thereon the computer program.

### BRIEF DESCRIPTION OF THE DRAWINGS

Non-limiting examples will now be described in reference to the accompanying drawings, where:
- FIGs. 1-2 show flowcharts of examples of the method;
- FIGs. 3-13 illustrate example implementations of the method; and
- FIG. 14 shows an example of the system.

### DETAILED DESCRIPTION

It is proposed a computer-implemented method for predicting a hemodynamic vascular behavior of a plurality of arteries of a patient with uncertainty quantification.

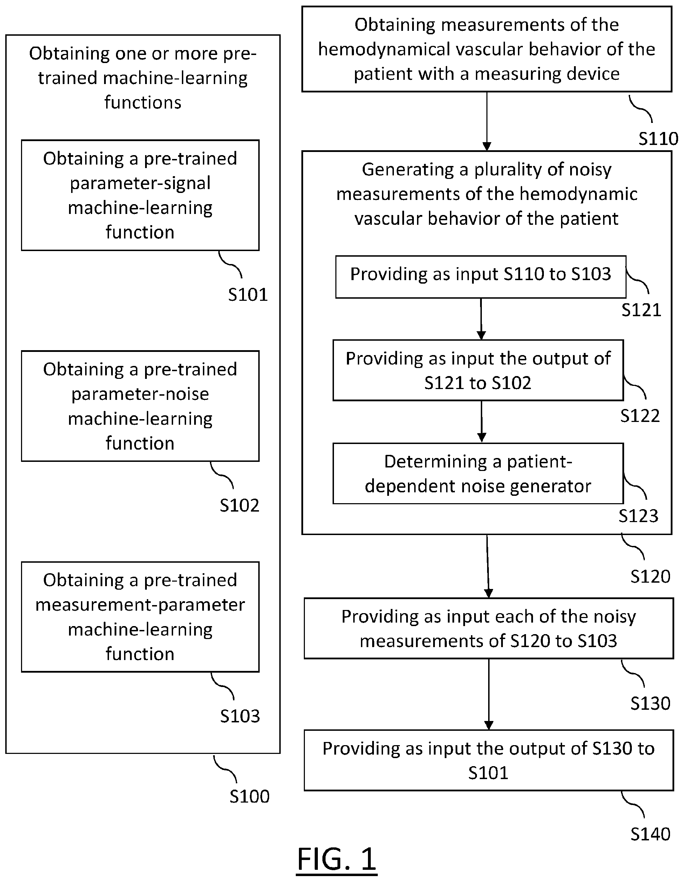

FIG. 1 illustrates a flowchart of the method, comprising a first step S100 of obtaining pre-trained machine-learning functions. S100 comprises three independent steps (S101, S102, and S103) of obtaining a respective pre-trained machine-learning function. The steps S101, S102, and S103 may be executed in any order in the method (e.g., in parallel or one after the other). The obtained pre-trained machine-learning functions are used in the right-part of the flowchart, consisting in steps each of which may depend on the previous one.

With reference to the flowchart of FIG. 1, the method comprises obtaining S100 one or more pre-trained machine-learning functions.

The obtaining of one or more pre-trained machine-learning functions comprises obtaining S101 a pre-trained parameter-signal machine-learning function (e.g., comprising one or more neural networks). The pre-trained parameter-signal machine-learning function has been trained for outputting an instance of predetermined hemodynamic signals from an instance of predetermined parameters provided as input. Wherein the predetermined hemodynamic signals comprise one or more hemodynamic signals. Each of the hemodynamic signals characterizes a blood flow of a respective artery of the plurality of arteries of the patient. Wherein the predetermined parameters comprise one or more parameters. Each of the parameters characterizes a respective artery of the plurality of arteries of the patient. The obtaining of one or more pre-trained machine-learning functions further comprises obtaining S102 a pre-trained parameter-noise machine-learning function (e.g., comprising one or more neural networks). The pre-trained parameter-noise machine-learning function has been trained for outputting an instance of predetermined characterizations of hemodynamic localization noise from an instance of the predetermined parameters provided as input. Wherein the predetermined characterizations of hemodynamic localization noise comprise one or more characterizations of noise (e.g., one or more covariance matrices). Each characterization of noise characterizes a variability of a hemodynamic signal characterizing a blood flow of a respective artery of the plurality of arteries of the patient. The variability of the hemodynamic signal characterizing the blood flow is with respect to a location in the artery. The obtaining of one or more pre-trained machine-learning functions further comprises obtaining S103 a pre-trained measurement-parameter machine-learning function (e.g., comprising one or more neural networks). The pre-trained measurement-parameter machine-learning function has been trained for outputting an instance of the predetermined parameters from measurements of the hemodynamic vascular behavior provided as input. Wherein the measurements of the hemodynamic vascular behavior comprise instances of hemodynamic signals characterizing the blood flow of a predetermined subset of arteries of the plurality of arteries of the patient.

The method further comprises obtaining S110 measurements of the hemodynamical vascular behavior of the patient with a medical measuring device. The method further comprises generating S120 a plurality of noisy measurements of the hemodynamic vascular behavior of the patient.

The generating S120 of a plurality of noisy measurements comprises providing S121 as input the obtained measurements of the hemodynamic vascular behavior of the patient to the pre-trained measurement-parameter machine-learning function. Thereby the method obtains a patient-dependent instance of the predetermined parameters. The generating of a plurality of noisy measurements further comprises providing S122 as input the obtained patient-dependent instance of the predetermined parameters to the pre-trained parameter-noise machine-learning function. Thereby the method obtains a patient-dependent instance of the predetermined characterizations of hemodynamic localization noise. The generating of a plurality of noisy measurements further comprises determining S123 a patient-dependent noise generator from the patient-dependent instance of the predetermined characterizations of hemodynamic localization noise. The patient-dependent noise generator is able to modify the measurements of the hemodynamic vascular behavior of the patient by adding noise. The noise quantifies possible errors in the measurements of the hemodynamic vascular behavior of the patient with the medical measuring device.

The method further comprises providing S130 as input each of the noisy measurements of the hemodynamical vascular behavior of the patient to the pre-trained measurement-parameter machine-learning function. The method thereby obtains a plurality of noisy patient-dependent instances of the predetermined parameters. The method further comprises providing S140 as input each of the obtained plurality of noisy patient-dependent instances of the predetermined parameters to the pre-trained parameter-signal machine-learning function. The method thereby obtains a plurality of noisy patient-dependent instances of the predetermined hemodynamic signals.

The method thereby constitutes an improved solution to predicting a hemodynamic vascular behavior of a plurality of arteries of a patient. The method can be used, for example, by a health specialist to obtain predictions of hemodynamic signals (e.g., blood pressure, flow rate, cross-sectional area, or blood velocity) of arteries that, e.g., are not easily accessible with medical devices (e.g., not accessible with non-intrusive measures). Indeed, the health specialist can obtain measurements of the hemodynamical vascular behavior of a patient with a medical measuring device of accessible arteries (e.g., arteries in arms and/or legs) and the method can predict the hemodynamic signals of arteries that are not easily accessible with medical devices (e.g., arteries in the brain) based on the obtained measurements. In examples, the method can only (e.g., exclusively) need measurements of the hemodynamical vascular behavior of a patient with non-intrusive methods (e.g., with a non-intrusive medical measuring device). This facilitates the application of the method by removing the need for expensive, time-consuming, and/or intrusive measurements. Indeed, thanks to the method, a health specialist can obtain information about the blood flow in arteries that are not directly assessable with non-intrusive methods.

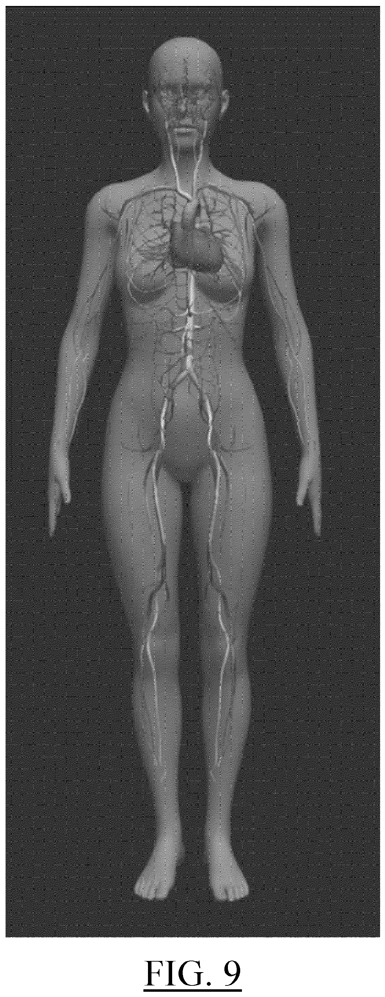

Prior art methods for predicting a vascular behavior of a patient either lack mechanisms for estimating the inherent uncertainties present in each simulation model, or rely on computationally intensive techniques that must be adapted to each case. Therefore, a health specialist, e.g., a physician, while using prior art methods, may not know how trustworthy the obtained information is. This is a big limitation, making the prior art methods only capable of giving an indication of the patient's condition without being reliable sources of information. On the contrary, the method of the current disclosure can be used to quantify/estimate the uncertainties (i.e., the confidence level) of the predicted hemodynamical signals at minimal computational cost for each new patient. The method can, for example, be used to quantify/estimate the uncertainties coming from the measurements of the hemodynamic behavior of a patient due, e.g., to a localization error in the measurements. Thereby, a health specialist using the method, can be able to better assess the situation of the patient. Indeed, the health specialist can estimate with the method the confidence they can have on the predicted perfusion values. For example, the health specialist can estimate the worst-case scenario and advise the patient in relation to this worst-case scenario. For example, whenever the worst-case scenario is acceptable (e.g., not dangerous, healthy), the health specialist can assert with confidence that the condition of the patient is healthy/not dangerous. The method was implemented, and a description of an example of the implementation and the use of the example of the implementation is given below in the disclosure with reference to FIGs. 9-13.

The method may comprise customizing a digital twin (or virtual twin) of the patient. In this disclosure, any digital twin of a patient may be a virtual representation (i.e., "digital twin") of the patient (i.e., "physical twin"). Digital twins may rely on model-based simulations of complex physics phenomena that may be parametrized to fit the patient's specificities. A digital twin may serve as a tool for medical applications, such as preoperative planning, procedural simulation, educational purposes, or the development and testing of medical devices tailored to the patient's specific vascular geometry and flow dynamics. As cardiovascular diseases are the number one cause of death globally, digital twins can be an enabling tool for precision medicine, by providing a personalized virtual representation of the patient and their pathologies. Digital twins may be used for the diagnosis and treatment of cardiovascular pathologies by simulating the way blood flows in the arterial and venous systems.

The customizing of a digital twin of the patient may comprise setting parameters (e.g., instances of the predetermined parameters) and/or signals (e.g., instances of the predetermined hemodynamic signals) in the digital twin. Therefore, the method enables the customizing of a digital twin of a patient starting from the obtaining S110 of measurements of the hemodynamical vascular behavior of a patient with a medical measuring device (e.g., with a non-intrusive device). Thereby the digital twin may be customized to the specific case of the specific patient. Such a patient-specific adaptation improves the precision of the digital twin. The method enables the customization of a digital twin of a patient with quantified uncertainties. That is, the hemodynamic signals comprised (e.g., shown in a graphical interface) in a digital twin obtained with the method may comprise interval of confidence or reliability values. As explained above, the quantified uncertainties further increase the reliability of the digital twin, thereby increasing its usability.

The method may comprise a graphical representation of a digital twin. The graphical representation may, for example, be displayed on a computer screen or the like. The graphical representation may comprise a summary of the physiological parameters (e.g., comprising the predetermined parameters) and/or the hemodynamical signals (e.g., comprising the predetermined hemodynamic signals) of the patient. The graphical representation may comprise the uncertainties of some (e.g., each) of the shown physiological parameters and/or hemodynamical signals. The summary may also comprise the physiological features of a patient, like name, or age.

In the present disclosure, a digital twin may comprise a digital representation of the patient's vascular system (e.g., a vascular model), that may include arteries, veins, and/or capillaries (e.g., only arteries), designed to replicate the unique anatomical and physiological characteristics of the patient. The digital representation is a virtual model made by a computer based on, partly or totally, real-life data of the patient's vascular system. The vascular model may comprise a plurality of sub-models. Each sub-model may comprise and be defined by a subset of the predetermined parameters. In examples, each of the sub-models may be with respect to an artery of the plurality of arteries and may be configured to simulate the behavior of the said artery. In examples, the sub-models may be connected according to a vascular graph which defines the relation between outputs and inputs of the sub-models. In examples, the vascular model may be a one-dimensional graph-like structure, decomposed into a series of arterial segments connected by junction and bifurcation nodes. Each vessel may be modeled by an axisymmetric deformable tube, with a time-varying radius and quantities of interest that are described by the tube's single axial coordinate.

Predicting a hemodynamic vascular behavior of a plurality of arteries of a patient (e.g., for customizing a vascular model and/or digital twin) and quantifying the uncertainties involve tuning the predetermined parameters and determining an instance of the hemodynamic signals. Such operations may be computationally highly expensive, in terms of CPU consumption and memory use.

For example, the predetermined parameters may comprise 300 or more parameters and the calibration of vessel parameters is often performed through an inverse problem. Indeed, it may not be possible (e.g., at all, or without intrusive measures) to determine with a medical measure device each of the (predetermined) parameters. The measurements (e.g., obtained with medical measuring devices) may be sparse, noisy, and difficult to acquire. Moreover, the inverse problem (e.g., used to calibrate the predetermined parameters) may be ill-posed, that is it may not have an exact solution, or it may have several solutions. The method, using a combination of Bayesian inference and pre-trained machine-learning functions (obtained at S100), is able to quickly and reliably solve the inverse problem, thereby being able to calibrate the predetermined parameters to measurements of a patients in real time.

Specifically, the anatomical or geometrical parameters (lengths, radii) may be measured through various imaging techniques, with varying levels of intrusiveness. For example, the anatomical or geometrical parameters may be measured with Magnetic Resonance Angiography (MRA), that is not invasive compared to its catheter counterpart. MRA is a type of Magnetic Resonance Imaging (MRI), that does not require radiation, and that looks specifically at the body's blood vessels. MRA is usually used to measure the neck, brain, abdominal and peripheral arteries (arms and legs). For example, the anatomical or geometrical parameters may be measured with brightness-mode (B-mode) ultrasonography, also known as grayscale ultrasound. B-mode ultrasonography is a non-intrusive technique that interrogates tissues via highfrequency sound waves. It may be used for anatomical localization and vessel size measurements. However, the terminal model parameters (e.g., the Windkessel model parameters) are usually obtained through inverse techniques, that is, from an instance of the predetermined hemodynamic signals. For example, the anatomical or geometrical parameters may be measured with catheter angiography, that is, with a visualization of the lumen (inside of blood vessels). Catheter angiography is an intrusive technique that uses a catheter, X-ray imaging guidance, and the injection of radio-opaque contrast agent into the blood vessel.

In the method of the present disclosure, the parameters are not directly obtained with any of the above measuring medical devices, as they are obtained through one or more of the pre-trained machine-learning functions obtained at S100, for example with the measurement-parameter machine-learning function obtained at S103. Therefore, the method allows the obtaining of instances of the predetermined parameters of the patient without any intrusive measure.

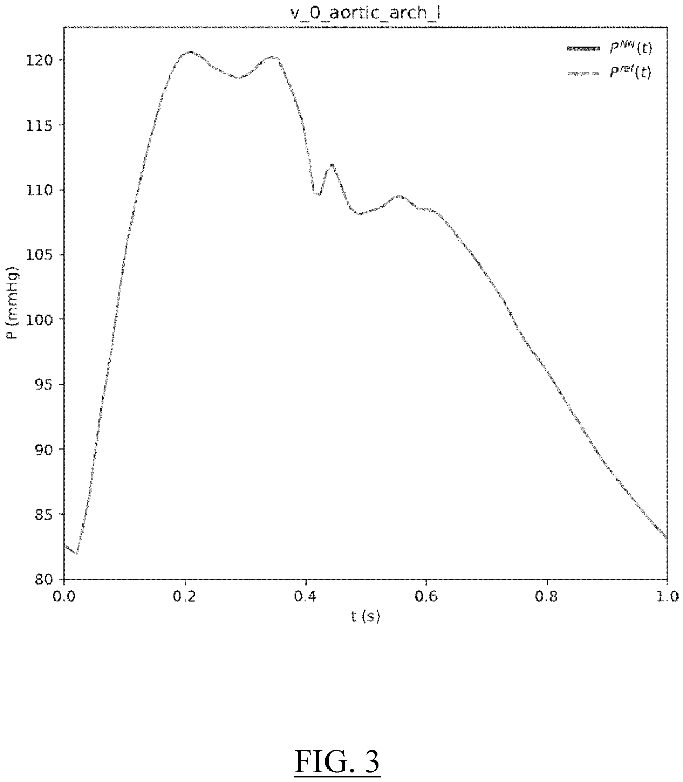

Once the predetermined parameters are calibrated, determining an instance of the predetermined hemodynamic signals may also be computationally expensive. For example, in prior art methods the customizing of a vascular model and/or digital twin and the simulating of the hemodynamic vascular behavior of the plurality of arteries involved solving complex equations, taking a long time (e.g., several minutes). Indeed, high-fidelity computational hemodynamics simulations in three dimensions (3D) are typically very expensive computationally (e.g., in terms of resources and time). The method, thanks to the pre-trained machine-learning functions obtained at S100, is able to customize the vascular model and/or digital twin and simulate the blood flow in real time, thereby enabling the manipulating/customizing a vascular model of a patient in real time. The reliability of the machine-learning functions used in the method can be, e.g., visually observed in FIG. 3 and in FIGs. 9-13 describing the implementation. The figures are described below in the disclosure. Herein, by real-time it is meant that the predictions can be obtained after a few seconds, or after a few fractions of a second, after the obtaining S110 of measurements of the hemodynamical vascular behavior of the patient. More generally, real-time means that predictions can be obtained before a predetermined period of time has elapsed.

In this disclosure, hemodynamic vascular behavior pertains to the dynamics of blood flow within a circulatory system that may comprise temporal and/or spatial descriptions and representation of the physiological movement of blood in the system. The circulatory system comprises a plurality of arteries of a patient, said patient being a human or animal subject to examination (e.g., medical examination). The hemodynamic vascular behavior comprises one or more hemodynamic signals. That is, the method is for predicting one or more hemodynamic signals for one or more (e.g., each) of the arteries comprising the plurality of arteries of the patient. Each of the hemodynamic signals may quantify a blood flow in a respective artery. Each of the hemodynamic signals may be, but are not limited to, a blood pressure, a blood flow rate, a cross-sectional area, or a blood velocity of a respective artery. Each of the hemodynamic signals may depend on a time variable and/or a spatial variable. The time variable may be with respect to the heartbeat and may be normalized (e.g., between 0 and 1). The spatial variable may be a position in the respective artery and may also be normalized. In examples, each of the predicted hemodynamic signals may be always with respect to a single spatial point of a respective artery, e.g., the middle point of a respective artery. Therefore, in examples, each of the hemodynamic signals may quantify a blood flow at the middle point of a respective artery depending on a time variable. For example, a hemodynamic signal may describe a, e.g., blood pressure, blood flow rate, cross-sectional area, or blood velocity, at each time (with respect to the heartbeat) at the middle of a respective artery.

The plurality of arteries (two or more arteries) of the patient may be predetermined. The plurality of arteries may be any subset of arteries of a class of patients, e.g., a subset of the arteries of humans. In examples, the plurality of arteries may comprise the most important arteries of, e.g., humans. The most important may be with respect to the size and/or medical relevance.

The method further quantifies the uncertainties. In this disclosure, uncertainties refer to the potential variability or error in measurements, predictions, or calculations. Therefore, "with uncertainty quantification" means that the predicted hemodynamical vascular behavior of a plurality of arteries of a patient is given with statistical indicators including the potential variability or error in measurements and/or predictions and/or calculations. Uncertainty quantification (e.g., the statistical indicators) may comprise representing the range within which the true value is expected to lie with a specified level of confidence, encompassing both systematic and random sources that affect the reliability and accuracy of the results. Quantifying the uncertainties may increase the reliability of the predictions and take into account the effects of the variability, the randomness and the misunderstanding of the models, in order to assist in decision-making. The uncertainties may depend on aleatoric uncertainty, also known as stochastic uncertainty, which may be representative of unknowns that may differ each time that a same experiment is conducted. The uncertainties may also depend epistemic uncertainty, that is, the lack of knowledge or confidence in the model or the data. The uncertainties may further depend on measurements that may be intrinsically imperfect and subject to errors (e.g., due to human error using a medical measuring device, different human anatomies, or the errors of the medical measuring devices themselves).

The method comprises obtaining S100 one or more pre-trained machine-learning functions. Each of the machine-learning functions is a supervised model that is pre-trained on a respective training dataset. Each of the machine-learning functions may comprise (e.g., consists of) one or more neural networks, e.g., several neural networks.

As is known *per se* from machine-learning, a neural network may be defined by its architecture, parameters, and hyperparameters. The architecture consists of layers, starting with the input layer whose neuron count may be determined by the dimensionality of the input data. The input layer may be followed by several hidden layers with a given number of neurons and activation functions. These layers and neurons define the network's depth and width, while the activation functions may introduce non-linearity into the model. The output layer may have as many neurons as the variables in the output data. The interconnections between these layers define the topology of the neural network. The parameters of the neural network are the learnable weights and biases, which are determined in the training process. In contrast, the hyperparameters are pre-defined settings that are not learned from the training data. These encompasses the number of hidden layers, neurons per layer and much more.

The obtaining S100 may comprise training one or more machine-learning function. Training a machine-learning function (that may also be called, a model) comprises obtaining a training dataset and training the machine-learning function based on a training dataset. As is known *per se* from the field of machine-learning, the processing of an input by a model includes applying operations to the input, the operations being defined by data including weight values or parameters. Learning a model (e.g., a machine learning function) thus includes determining values of the weights/parameters based on a dataset configured for such learning, such a dataset being possibly referred to as a learning dataset or a training dataset. For that, the dataset includes data pieces each forming a respective training sample or training example. The training samples/examples represent the diversity of the situations where the model is to be used after being learnt. Any training dataset herein may comprise a number of training samples/examples higher than 5000, or 10000. In the context of the present disclosure, by "learning a model/machine-learning function based on a dataset", it is meant that the dataset is a learning/training dataset of the model/machine-learning function, based on which the values of the weights/parameters are set. In the present disclosure, the training dataset is the obtained dataset of training examples, on which the model is learnt/trained.

Each of the machine-learning functions may comprise (e.g., consists of) one or more neural networks, e.g., several neural networks. Therefore, training a machine-learning function may comprise training one or more neural networks. To train a neural network, at least two settings may be defined. First, a loss function, which is a metric that measures the error between the training data and the model's prediction, such as the mean square error or mean absolute error. Second, an optimizer, which modifies the model's weights and biases during the training process to minimize the loss function. Each optimizer has its own set of hyperparameters.

The obtaining S100 comprises obtaining S101 a pre-trained parameter-signal machine-learning function having been trained for outputting an instance of predetermined hemodynamic signals from an instance of predetermined parameters provided as input.

The predetermined hemodynamic signals comprise one or more hemodynamic signals, each of the hemodynamic signals characterizing (e.g., giving a quantitative description) a blood flow of a respective artery of the plurality of arteries of the patient. As explained above, each of the hemodynamic signals may depend on a time variable (that may be normalized and may depend on the heartbeat) and/or on a spatial variable (a position within the artery). The spatial variable may be fixed (e.g., a middle point of the artery). Each of the hemodynamic signals may be, e.g., blood pressure, blood flow rate, artery cross-sectional area, or blood velocity of a respective artery (e.g., at the middle point of the respective artery). An instance of the predetermined hemodynamic signals comprises (e.g., consists of) an instance (e.g., a numerical value and/or function) of each of the hemodynamic signals comprised in the predetermined hemodynamic signals. In examples, the predetermined hemodynamic signals may comprise one or more hemodynamic signals for each of the arteries of the plurality of arteries of the patient, the hemodynamic signal characterizing said artery. In examples, the predetermined hemodynamic signals may comprise (or consists of), e.g., a blood pressure and a blood flow rate for each of the artery comprised in the plurality of arteries (e.g., at the middle point of the artery). In other examples, the predetermined hemodynamic signals may comprise (or consist of), e.g., an artery cross-sectional area and blood velocity for each of the artery comprised in the plurality of arteries (e.g., at the middle point of the artery).

The predetermined parameters comprise one or more parameters, each of the parameters characterizing a respective artery of the plurality of arteries of the patient. Each of the parameters may give a quantitative description (e.g., with respect to a size, or an elasticity) of a respective artery. Each of the parameter comprised in the predetermined parameters may be an anatomical (e.g., a length, a radius), a physiological or physical (e.g., vessel stiffness), or a model (e.g., for the lumped model) parameter of a respective artery of the plurality of arteries of the patient. In examples, each of the parameter comprised in the predetermined parameter may be a Young's modulus, mass density of blood, dynamic viscosity, a radial profile of the longitudinal velocity, a vessel stiffness, a vessel length, a vessel radius (e.g., proximal and/or distal), a thickness of a vessel wall, a Poisson ratio, a rest cross-sectional area, a 0D Windkessel model parameter of a respective artery. In examples, the predetermined parameters may comprise a Young's modulus, a vessel length, a vessel radius (e.g., proximal and/or distal), a thickness of a vessel wall for each of the artery of the plurality of arteries. In examples, the predetermined parameters may further comprise the 0D Windkessel model parameters for each of the terminal arteries (i.e., the arteries that are leaves in the tree of arteries, arteries that are not connected downstream to any other artery). An instance of the predetermined parameters comprises (e.g., consists of) an instance (e.g., a numerical value and/or function) of each of the parameters comprised in the predetermined parameters.

The pre-trained parameter-signal machine-learning function obtained at S101 may comprise a pre-trained parameter-signal neural network model for one or more (e.g., each) of the hemodynamic signals of respective arteries of the plurality of arteries of the patient comprised in the predetermined hemodynamic signals. Each of the pre-trained parameter-signal neural network models may have been trained for outputting an instance of the respective hemodynamic signal of the respective artery from an instance of the predetermined parameters provided as input. That is, the pre-trained parameter-signal machine-learning function obtained at S101 may comprise one or more (e.g., one for each hemodynamic signal comprised in the predetermined hemodynamic signals) pre-trained parameter-signal neural network models, each of the pre-trained parameter-signal neural network models having been trained for outputting an instance of a respective hemodynamic signal from an instance of the predetermined parameters provided as input. Having a different pre-trained parameter-signal neural network for one or more (e.g., each) of the hemodynamic signals may be advantageous as each of the pre-trained parameter-signal neural networks may be faster and more efficient compared to a model trained to output all the hemodynamic signals altogether.

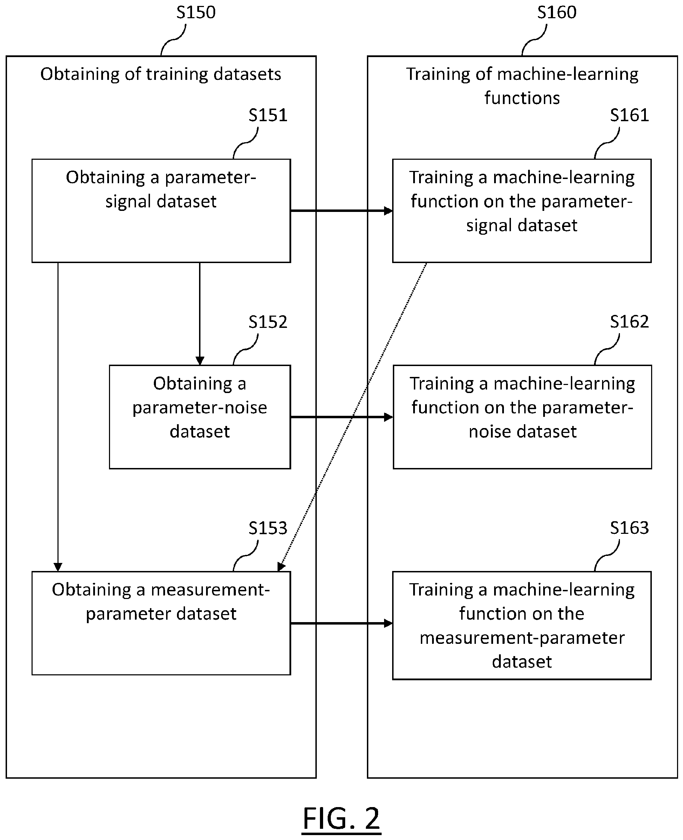

The flowchart of FIG. 2 illustrates two steps that may be comprised in the method: the obtaining S150 of training datasets and the training S160 of machine-learning functions. The obtaining S150 may comprise three different obtaining steps S151, S152, and S153 as described hereinafter. The training S160 may comprise three different training steps S161, S162, and S163 as described hereinafter. The (training) dataset that may be obtained at S151/2/3 may be used to train at S161/2/3 a respective machine-learning function. The trainings S161, S162, S163 are independent of each other, however, the machine-learning function trained at S161 may be used in the obtaining S153. The training dataset obtained at S151 may be used to obtained the datasets at S152 and S153.

With reference to the flowchart of FIG. 2, the obtaining S101 of the pre-trained parameter-signal machine-learning function may comprise obtaining S151 a parameter-signal dataset. The parameter-signal dataset may comprise entries, each entry comprising an instance of the predetermined parameters and an instance of the predetermined hemodynamic signals.

The obtaining S151 of a parameter-signal dataset may comprise generating the dataset with data of virtual patients. Generating a dataset with data of virtual patients may be beneficial, as the generated dataset may be tailored for training the required machine-learning function. The dataset of virtual patients may be created using reliable vascular models for simulating a blood flow, so as to obtain reliable data to be used in the training.

The obtaining S151 may comprise obtaining numerical bounds for each parameter comprised in the predetermined parameters. Each of the parameters may give, as described above, a quantitative description of a respective artery. Each of the numerical bounds may be related to and/or determine a physiological plausible range of the possible values (i.e., instances) of the respective parameter in a population. In examples, for each given parameter, the numerical bounds may comprise an upper bound and a lower bound. The physiological plausible range of possible values may consist of all the values in between the lower and upper bound. In examples, the physiological plausible range of the possible instances of a given parameter may be the smallest interval (e.g., comprised between a lower and upper bound) comprising all the instances/values of the given parameter in a population. In other words, in examples, the lower bound may be the lowest value/instance of the given parameter among all the patients in a population. Similarly, the upper bound may be the highest value/instance of the given parameter among all the patients in a population. Alternatively, in examples, the range of the possible instances of a given parameter may be the smallest interval comprising a percentage of all the instances/values of the given parameter in a population. The percentage may be, e.g., 90% or 95%. The numerical bounds for a given parameter may, therefore, express the range of possible values that the given parameter may take on each of the patients in the population. The population may be, e.g., all humans, all human adults, or all human children.

In formulae, the obtaining S151 may comprise for each of the predetermined parameters *k* obtaining a lower bound *aₖ* and an upper bound *bₖ* so that, e.g., all or most of the instances of the parameter k of the patients in the population are between *aₖ* and *bₖ.*

The obtaining S151 may comprise generating a parameter-list of instances of the predetermined parameters. Each instance may comprise an instance of each of the parameters comprised in the predetermined parameters. The instance of each of the parameters may be generated randomly within the obtained numerical bounds. By randomly generating, it is meant that the instance of a given parameter may be chosen uniformly at random (e.g., with respect to a Lebesgue measure) in the physiological plausible range associated to the given parameter. For example, the instance may be chosen uniformly at random between a lower bound and an upper bound.

The obtaining S151 may further comprise, obtaining for each instance of the predetermined parameters of the generated parameter-list, an instance of the predetermined hemodynamic signals by using a one-dimensional blood flow modeling solver. Thereby, the obtaining S151 may comprise determining entries of the parameter-signal dataset, each of the entry comprising an instance of the predetermined parameters and the associated obtained instance of the predetermined hemodynamic signals.

One-dimensional blood flow modeling solver that may be used in the method are herein discussed. As high-fidelity computational hemodynamics simulations in three dimensions (3D) are computationally expensive, the method may use one-dimensional (1D) and zero-dimensional (0D) models. Such one/zero-dimensional (1D/0D) models provide enough precision for medical use with a fraction of the computing cost of the high-fidelity models. In the model, the assumption that the blood is an incompressible Newtonian fluid may be made. The one-dimensional continuity and momentum conservation equations may be derived by integrating the Navier-Stokes equations along the radial and azimuthal coordinates of the vessel. The one-dimensional blood flow modeling solver may comprise the following formula: $\left\{\begin{matrix}\frac{\partial A}{\partial t} + \frac{\partial Q}{\partial x} = 0 \\ \frac{\partial Q}{\partial t} + \frac{\partial }{\partial x} \left(α\frac{{Q}^{2}}{A}\right) + \frac{A}{ρ} \frac{\partial P}{\partial x} = - 2 \frac{μ}{ρ} \left({γ}_{ν}+2\right) \frac{Q}{A}\end{matrix}\right.$

In the above formula, *t* is the time coordinate, *x* is the position within the artery, the quantity *Q*(*x, t*) is the blood flow rate, the quantity *P*(*x, t*) is the blood pressure, and the quantity *A*(*x, t*) is the artery cross-sectional area. Moreover, *α* is a parameter that reflects the radial profile of the longitudinal velocity (e.g., *α* ≈ 1.1, or *α =* 1.1), *ρ* is the mass density, *µ* is the dynamic viscosity, and ${γ}_{ν} = \frac{\left(α-2\right)}{1 - α}$. The one-dimensional blood flow modeling solver may further comprise the following formula: $\left\{\begin{matrix}P \left(A\right) = {P}_{ext} + β \left(\sqrt{\frac{A}{{A}_{0}}}-1\right) \\ β = \sqrt{\frac{π}{{A}_{0}}} \frac{{Eh}_{0}}{1 - {ν}^{2}}\end{matrix}\right.$

The above formula may be the constitutive elastic tube law equation constituting a closure equation between the pressure *P* and the cross-sectional area *A.* Moreover, *Pₑₓₜ* is the external pressure, *E* is the Young's modulus, v is the Poisson ration, *h*₀ is the thickness of the vessel wall, and *A*₀ is the rest cross-sectional area.

In examples, the obtaining S151 may therefore comprise, for each instance comprised in the parameter-list, using said instance of the predetermined parameters as input for the above-described one-dimensional blood flow modeling solver, based on the above-described formulas, thereby obtaining an instance of the predetermined hemodynamic signals. Each entry of the parameter-signal dataset may comprise an instance comprised in the parameter-list and the associated instance of the predetermined hemodynamic signals, e.g., obtained with the above formulas.

In examples, the output of the above-described one-dimensional blood flow modeling solver may be an instance of a hemodynamic signal depending both on a temporal (e.g., a time with respect to a heartbeat) and spatial a coordinate (e.g., a position in the artery). Such a hemodynamic signal depending both on temporal and spatial coordinate may be, in examples, called an extended hemodynamic signal. Thus, the output of the described one-dimensional blood flow modeling solver may be an instance of the extended hemodynamic signals. In examples, obtaining an instance of the predetermined hemodynamic signals may comprise restricting the spatial coordinate of an instance of the extended hemodynamic signals to a specific point (e.g., the middle point) of each of the arteries, that is, considering each of the solutions of the blood solver only at the specific point (e.g., the middle point) of the respective artery. In examples, each entry of the parameter-signal dataset may further comprise an instance of the extended hemodynamic signals associated to the instance of the predetermined parameter of the entry.

Therefore, in examples, the obtaining S151 may comprise obtaining for each instance of the predetermined parameters of the generated parameter-list an instance of the extended hemodynamic signals by using a one-dimensional blood flow modeling solver, the instance depending both on a temporal and spatial coordinate. The obtaining S151 may further comprise, for each instance of hemodynamic signals depending both on a temporal and spatial coordinate, restricting the instance to the middle point of the respective artery. In particular, each entry of the parameter-signal dataset may comprise an instance of the predetermined parameters, an instance of the extended hemodynamic signals, and an instance of the predetermined hemodynamic signals. The instance of the extended hemodynamic signals may be obtained by using a one-dimensional blood flow modeling solver and the instance of the predetermined hemodynamic signals by restricting the instance of the extended hemodynamic signals to a location in the artery.

In examples, the obtaining S151 may comprise generating a list of virtual patients. The list may represent a synthetic population of patients. Each of the virtual patients may comprise a generated ID, name, age, and sex (e.g., part of the comprised data may be randomly generated). In examples, the obtaining S151 may further comprise, for each virtual patient, generating an instance of the predetermined parameters, that is, an instance of each of the parameters comprised in the predetermined parameters. The generating may be at random within numerical bounds, as explained above. In examples, the obtaining S151 may further comprise, for each virtual patient, obtaining/determining an instance of the predetermined hemodynamic signals by using a one-dimensional blood flow modeling solver. Thereby, each virtual patient may be associated with an instance of the predetermined parameters and the associated instance of the predetermined hemodynamic signals obtained by using a one-dimensional blood flow modeling solver. In formulae, the list of virtual patients may form the database: $D = \left\{\left({p}^{\left(1\right)}, {S}^{\left(1\right)}\right),…,\left({p}^{\left(N\right)}, {S}^{\left(N\right)}\right);{p}^{\left(i\right)}∈{ε}_{p},{S}^{\left(i\right)}=F\left({p}^{\left(i\right)}\right)∈{ε}_{S}\right\}$ where *p*^{(*i*)} is an instance of the predetermined parameters (e.g., comprising Young's modulus, vessel lengths and radii, 0D Windkessel model parameters of each of the arteries of the plurality of arteries), *ε*ₚ is the space of all the possible instances of the predetermined parameters, *ε_{S}* is the space of all the possible instances of the predetermined hemodynamic signals, and *S*^{(*i*)} = *F*(*p*^{(*i*)}) is the instance of the predetermined hemodynamic signals associated with the instance *p*^{(*i*)} of predetermined parameters. Herein, the function *F* may indicate the solution obtained by using a one-dimensional blood flow modeling solver, e.g., the above-described one-dimensional blood flow modeling solver based on the above-described formulae.

The obtaining S151 may further comprise normalizing each of the instances of the parameters comprised in the instances of the predetermined parameters comprised in the generated list and each of the instances of the hemodynamic signals comprised in the obtained instances of predetermined hemodynamic signals. In other words, the obtaining S151 may further comprise normalizing each instance of the parameters and/or hemodynamic signals comprised in each entry of the parameter-signal dataset. In examples, the obtaining may comprise normalizing the instances of the virtual patients. Normalizing may be relevant for training the machine-learning functions, as input and output data are of various scales and physical nature, ranging from 10⁻⁹ to 10¹¹. Data normalization, or feature scaling, may be important in order to give each variable equal weights/importance during the L2 optimization process.

In examples, normalizing each of the instances of a respective parameter comprised in the instances of the predetermined parameters of the generated parameter-list may comprise reducing to the unitary hyper-cube based on the variation range (i.e., the physiological plausible range) computed for the input space sampling: $∀ k ∈ \left〚1, {N}_{p}\right〛 , {p}_{k} ∈ \left[{a}_{k}, {b}_{k}\right] \to {p}_{k}^{′} = \frac{{p}_{k} - {a}_{k}}{{b}_{k} - {a}_{k}} ∈ \left[0, 1\right]$ where *k* is a parameter comprised in the *Nₚ* predetermined parameters, *aₖ* is the obtained lower bound for the parameter *k* and *bₖ* is the obtained upper bound for the parameter *k*.

In examples, normalizing each of the instances of the hemodynamic signals comprised in the instances of the predetermined hemodynamic signals comprised in the entries of the parameter-signal dataset may comprise standardizing the instance through their mean and/or standard deviation value. In examples, the normalizing may comprise using the following formulae: $\overline{S} \left(t\right) = {E}_{p} \left[S\left(t, p\right)\right] ≡ \frac{1}{N} {\sum }_{i = 1}^{N} {S}_{i} \left(t\right)$ $S {"}_{i} \left(t\right) = \frac{{S}_{i} \left(t\right) - \overline{S} \left(t\right)}{A}$ ${A}^{2} = \frac{1}{N} {\sum }_{i = 1}^{N} {\left‖\left({S}_{i}\left(t\right)-{\overline{S}}_{i}\left(t\right)\right)\right‖}_{T}^{2} = \frac{1}{N} {\sum }_{i = 1}^{N} \left(\frac{1}{{N}_{T}}{\sum }_{k = 1}^{{N}_{T}} {\left({S}_{i}\left({t}_{k}\right)-\overline{S}\left({t}_{k}\right)\right)}^{2}\right)$ $\frac{1}{N} {\sum }_{i = 1}^{N} {\left‖S{"}_{i}\left(t\right)\right‖}_{T}^{2} = \frac{1}{N} {\sum }_{i = 1}^{N} \left(\frac{1}{{N}_{T}}{\sum }_{k = 1}^{{N}_{T}} {\left(S{"}_{i}\left({t}_{k}\right)\right)}^{2}\right) = 1$ where:
∘ *Sᵢ*(*t*) is the signal of the *i*-th artery,
∘ *tₖ* for *k =* 1, *... , N_{T}* is a predetermined discretization of the time variable *t,* such discretization may optionally be simplified (e.g., if the simulation returns 1000 time steps, the method may comprise only considering 100 time steps on the same grid), and
∘ ${S}_{i}^{"} \left(t\right)$ is the adimensional version of the signal *Sᵢ* of the *i*-th artery.

The obtaining S101 of the pre-trained parameter-signal machine-learning function may further comprise training S161 a machine-learning function on a parameter-signal dataset, thereby obtaining the pre-trained parameter-signal machine-learning function. The parameter-signal dataset may be the dataset obtained at S151 and may be obtained according to the above example. The efficacy of the machine learning function may be visually seen in FIG. 3 described hereinafter.

In examples, the machine-learning function to be trained at S161 may comprise, for one or more (e.g., for each) of the *N_{S}* hemodynamic signal comprised in the predetermined hemodynamic signals, a neural network. Therefore, in examples, the method may not use any correlation between the different hemodynamic signals. Each of the neural networks may comprise one or more layers (e.g., 4 layers), each layer consisting of a multitude of neurons (e.g., 50 neurons). Each of the neural networks may comprise an activation function (e.g., a Tanh function) and may have a final linear layer.

In examples, each of the neural network may be trained on the above defined parameter-signal dataset (e.g., the dataset 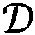 ) obtained at S151 consisting of randomly generated instances of the predetermined parameters and the associated instances of the predetermined hemodynamic signals obtained by solving the one-dimensional blood flow modeling solver, as described above. In examples, training each of the neural networks may comprise an optimization loss function 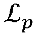 (*θ*) given by: ${L}_{p} \left(θ\right) = {E}_{p} \left[{\left‖\hat{F}\left(p, θ\right)-S\right‖}_{{ε}_{S}}\right] = {\int }_{{ε}_{p}} {\left‖\hat{F}\left(p, θ\right)-S\right‖}_{{ε}_{S}} {ℙ}_{p}$ where *F̂*(p,θ) is the result of the neural network with internal parameter (e.g., parameters as neural network) *θ*, *p* is an instance of the predetermined parameters, and *S* = *F*(*P*) is the instance of the predetermined hemodynamic signals associated with *p*.

In examples, training each of the neural networks may comprise splitting the training parameter-signal dataset (e.g., 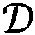 ) into a training and a testing dataset. The training may comprise computing the optimized loss function 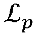 (*θ*) on the training dataset, and computing the validation error function 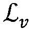 (*θ*) on the testing dataset, where the validation function may be given by the following formula: ${L}_{v} = \frac{\frac{1}{{N}_{v}} {\sum }_{i = 1}^{{N}_{v}} {\left‖S{"}_{i}-\hat{S}{"}_{i}\right‖}_{T}^{2}}{{{\frac{1}{{N}_{v}} {\sum }_{i = 1}^{{N}_{v}} {\left‖S{"}_{i}\right‖}_{T}^{2}}_{︸}}_{\approx 1}}$ where ${\hat{S}}_{i}^{"}$ is the normalized and/or adimensional version of the result of the neural network and *Nᵥ* is the size of the validation dataset. As the size of the global dataset is *N,* comprising both the validation and training dataset, *Nᵥ < N.* The sum $\frac{1}{{N}_{v}} {\sum }_{i = 1}^{{N}_{v}} {\left‖{S}_{i}^{"}\right‖}_{T}^{2}$ may not be exactly 1 as it is the sum over a smaller dataset (i.e., the validation dataset).

In examples, each of the neural networks may be trained according to the following:

| GPU | Architecture | Activation function | Optimizer | Learning rate | Number of epochs | Training time |
|---|---|---|---|---|---|---|
| NVIDIA A6000 | 4 layers of *Nₗ* = 50 neurons | Tanh with final linear layer | Adam | 1e-5 | ~ 500K | ~ 7200 s |

FIG. 3 illustrates two graphs describing blood pressure, dimensional and adimentional values P(t) (mmHg) in the aortic arch for a virtual patient of the testing dataset generated/obtained at S151. One graph *P^{NN}*(*t*) is the graph obtained with a trained parameter-signal neural network comprised in the parameter-signal machine-learning function trained at S161 predicting the blood pressure of the aortic arch. The other graph *P^{ref}*(*t*) is the ground truth used for training the machine-learning function (and hence the neural network), that is the graph obtained by solving the one-dimensional blood flow modeling solver and comprised in 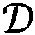 . The prediction error is lower or equal to 3% across all the time steps, and is consistent with the global validation loss 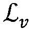 = 3%. That is, the output of the trained parameter-signal neural network is very closed to the ground truth, showing the efficacy of the method.

The obtaining S100 comprises obtaining S102 a pre-trained parameter-noise machine-learning function having been trained for outputting an instance of predetermined characterizations of hemodynamic localization noise from an instance of the predetermined parameters provided as input.

The predetermined characterizations of hemodynamic localization noise comprise one or more characterizations of noise, each characterization of noise characterizing (e.g., giving quantitative bounds) a variability of a hemodynamic signal characterizing a blood flow of a respective artery of the plurality of arteries of the patient. The variability being with respect to a location in the artery. In specific, a hemodynamic signal characterizing a blood flow may be dependent on a spatial coordinate (e.g., a position in the artery where the hemodynamic signal is consider, as explained above) and the each characterization of noise may characterize the variability (e.g., the dependence) of the hemodynamic signal characterizing the blood flow with respect to the spatial coordinate (e.g., the position in the artery). In examples, the hemodynamic signal characterizing the blood flow may be a blood pressure, blood flow rate, cross-sectional area, or blood velocity of the respective artery, said signal being considered at a specific point (e.g., the middle point) of the artery. The characterization of the noise may characterize/quantify the variability of the hemodynamic signal with respect to the position, that is variability between the hemodynamic signal at the specific point (e.g., the middle point) of the artery and nearby points in the artery. In examples, each of the characterizations of the noise may be a covariance matrix that may be used to define a multivariate normal distribution characterizing the variability of the hemodynamic signal with respect to the spatial coordinate.

In examples, the predetermined characterizations of hemodynamic localization noise may comprise one or more (e.g., one) characterizations of noise for one or more of the hemodynamic signals characterizing the blood flow of a predetermined subset of arteries of the plurality of arteries of the patient, each of the characterizations of noise characterizing (e.g., giving quantitative bounds) a variability of the respective hemodynamic signal. That is, in examples, each characterization of noise of the predetermined characterizations of hemodynamic localization noise may characterize a variability of a respective hemodynamic signal comprised in the hemodynamic signals characterizing the blood flow of a predetermined subset of arteries of the plurality of arteries of the patient. In examples, the predetermined characterizations of hemodynamic localization noise may, therefore, characterize a localization noise of the hemodynamic signals characterizing the blood flow of a predetermined subset of arteries of the plurality of arteries of the patient.

The pre-trained parameter-noise machine-learning function obtained at S102 may comprise a pre-trained parameter-noise neural network model for one or more (e.g., each) characterization of noise characterizing a variability of a hemodynamic signal characterizing a blood flow of a respective artery of the plurality of arteries of the patient comprised in the predetermined characterizations of hemodynamic localization noise. Each of said pre-trained parameter-noise neural network models may have been trained for outputting an instance of the characterization of noise of the respective artery from an instance of the predetermined parameters provided as input. That is, the pre-trained parameter-noise machine-learning function obtained at S102 may comprise one or more (e.g., one for each characterization of noise comprised in the predetermined characterizations of hemodynamic localization noise) pre-trained parameter-noise neural network models, each of the pre-trained parameter-noise neural network models having been trained for outputting an instance of the characterization of noise of the respective artery from an instance of the predetermined parameters provided as input. Having a different pre-trained parameter-noise neural network for one or more (e.g., each) of the characterization of noise may be advantageous as each of the pre-trained parameter-noise neural networks may be faster and more efficient compared to a model trained to output all the characterizations of noise altogether.

The obtaining S102 of the pre-trained parameter-noise machine-learning function may comprise obtaining S152 a parameter-noise dataset. The parameter-noise dataset may comprise entries, each entry comprising an instance of the predetermined parameters and an instance of the predetermined characterizations of hemodynamic localization noise.

The obtaining S152 of a parameter-noise dataset may comprise, generating the parameter-noise dataset using the dataset of virtual patients generated at S151. Using the dataset of virtual patients may be beneficial, as the generated dataset may be tailored for training the required machine-learning function.

The obtaining S152 may comprise, for each given entry of the parameter-signal dataset obtained at S151, obtaining an instance of the predetermined characterizations of hemodynamic localization noise. Thereby, the obtaining S152 may comprise determining entries of the parameter-noise dataset, each of the entry comprising an instance of the predetermined parameters comprised in a given entry of the parameter-signal dataset and the associated obtained instance of the predetermined characterizations of hemodynamic localization noise.

The obtaining S152 may comprise, for each given entry of the parameter-signal dataset obtained at S151, determining a covariance matrix of each of the instances of the hemodynamic signals associated to (i.e., comprised in) the given entry with respect to the localization. The given entry may comprise an instance of the predetermined hemodynamic signals and may also comprise an instance of the extended hemodynamic signals. Each of the hemodynamic signals associated to (i.e., comprised in) the given entry may be one of the instances of the predetermined hemodynamic signals or one of the instances of the extended hemodynamic signals. The covariance matrix of each of the instances of the hemodynamic signals may quantify a variability of the localization (e.g., a variability with respect to a spatial variable) of the instance of the respective hemodynamic signal.

In examples, each of the hemodynamic signals comprised in the predetermined hemodynamic signals may be with respect to a specific point (e.g., a middle point) of a respective artery. Therefore, the measurements of the hemodynamic vascular behavior obtained at S110 should be acquired at the associated specific point, such as middle point, or as close as possible to the specific point, of each of the measured artery. Since this exact localization is impossible to find when conducting a measure with a medical measuring device (e.g., with an ultrasound), all the measurements come with a localization error. Due to the non-linear wave propagation nature of the blood flow, the covariance of the modeled localization error is by nature heteroscedastic (meaning that the variance of the noise that is applied to a signal will vary over time).

In examples, the method may comprise a physics-based approach based on the simulation of the pulse wave propagation of the blood signal at *Nₓ* spatial points along the blood vessel of length L. That is, the method may comprise considering instances of hemodynamic signals at *Nₓ* different spatial points (e.g., location in the artery). In examples, the predetermined hemodynamic signals and the hemodynamic signals characterizing a blood flow of a respective artery of the plurality of arteries of the patient may be with respect to a middle point of the respective arteries. As stated above, all the measurements come with a localization error, and in examples, it may be assumed that the measurement of the vascular behavior of the patient are acquired between the ${x}_{1} = \frac{L}{4}$ and ${x}_{2} = \frac{3 L}{4}$ longitudinal coordinates, instead of the exact center of the vessel at ${x}_{c} = \frac{L}{2}$.

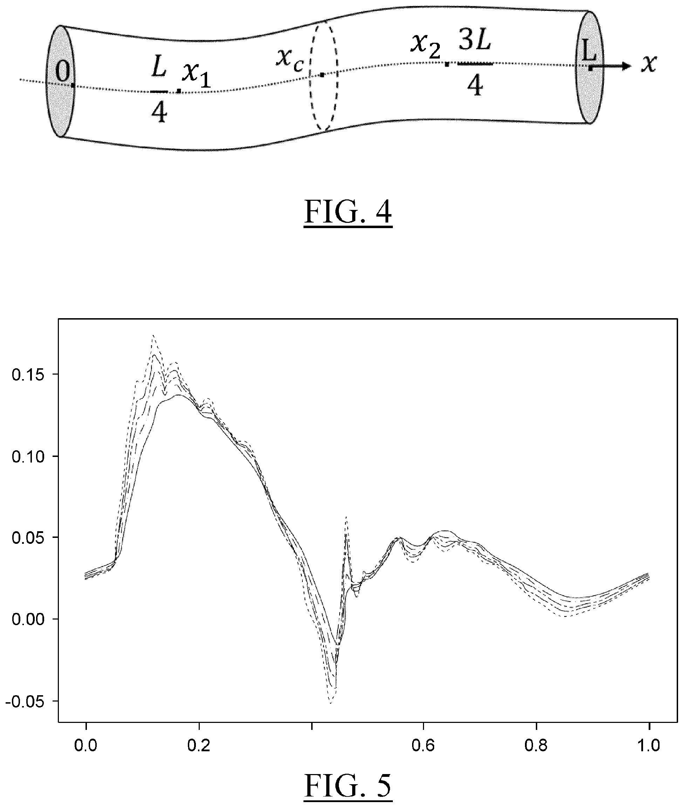

FIG. 4 illustrates a model of a vessel, with a center point *x_{c}* and the point *x*₁ = $\frac{L}{4}$ and ${x}_{2} = \frac{3 L}{4}$. The measurement of the vascular behavior of the patient may be acquired in an unknown location between *x*₁ and *x*₂, whereas the predetermined hemodynamic signals and the hemodynamic signals characterizing a blood flow of the artery may be with respect to *x_{c}.*

FIG. 5 shows examples of hemodynamic signals consisting of respective blood velocity at *Nₓ* = 5 (different) spatial points comprised in the interval $\left[\frac{L}{4}, \frac{3 L}{4}\right]$. FIG. 5 shows the possible amplitude of the localization error and highlights the non-linear propagation behavior of the pulse wave.

In examples, each of the instances of the hemodynamic signals comprised in a given entry of the parameter signal dataset (of the instances of the predetermined/extended hemodynamic signals) may be denoted *S*(*x*,*t*|*p*), where p may be the instance of the predetermined parameter comprised in the entry, x is a position relative to the artery, and *t* is the time variable. In examples, the covariance matrix of an instance of a hemodynamic signal *S*(*x,t*|*p*) may be determined with the formula: $\begin{matrix}{C}^{loc} \left(t,t′\left|p\right.\right) = {E}_{x} \left[\left(S\left(x,t\left|p\right.\right)-{E}_{x}\left[S\left(x,t\left|p\right.\right)\right]\right)\left(S\left(x,t′\left|p\right.\right)-{E}_{x}\left[S\left(x,t′\left|p\right.\right)\right]\right)\right] \\ = {E}_{x} \left[S\left(x,t\left|p\right.\right)S\left(x,t′\left|p\right.\right)\right] - {E}_{x} \left[S\left(x,t\left|p\right.\right)\right] {E}_{x} \left[S\left(x,t′\left|p\right.\right)\right]\end{matrix}$ where *t* and *t'* are time coordinates, x is the position within the artery (i.e., the spatial coordinate), and p is the instance of the predetermined parameters comprised in the entry.

In examples, determining a covariance matrix with the above formula may comprise, obtaining a set of *Nₓ > Nt* longitudinal coordinates that are uniformly drawn between $x = \frac{L}{4}$ and $x = \frac{3 L}{4}$ and *Nₜ* timesteps t: $x ∼ U \left[\frac{L}{4}, \frac{3 L}{4}\right] , \left\{{x}_{i},i=1,…,{N}_{x}\right\} .$

Determining a covariance matrix with the above formula may further comprise computing the expected values empirically on the sampled data: ${C}^{loc} \left(t,t′\left|p\right.\right) = \frac{1}{{N}_{x}} {\sum }_{i = 1}^{{N}_{x}} S \left({x}_{i},t\left|p\right.\right) S \left({x}_{i},t′\left|p\right.\right) - \frac{1}{{N}_{x}^{2}} \left({\sum }_{i = 1}^{{N}_{x}} S \left({x}_{i},t\left|p\right.\right)\right) \left({\sum }_{i = 1}^{{N}_{x}} S \left({x}_{i},t′\left|p\right.\right)\right)$

Therefore, in examples, the obtaining S152 may comprise, for each given entry of the parameter-signal dataset (e.g., obtained at S151), determining a covariance matrix with the above formula (e.g., computing the expected values empirically) of each of the instances of the hemodynamic signals comprised in the instances of the predetermined/extended hemodynamic signals.

The obtaining S102 of the pre-trained parameter-noise machine-learning function may further comprise training S162 a machine-learning function on a parameter-noise dataset, thereby obtaining the pre-trained parameter-noise machine-learning function. The parameter-noise dataset may be the dataset obtained at S152 and may be obtained according to the above example.

In examples, the machine-learning function to be trained at S162 may comprise, for one or more (e.g., for each) characterization of noise characterizing a variability of a hemodynamic signal, a neural network. Therefore, in examples, the method may not use any correlation between the different characterization of noise of the hemodynamic signals. Each of the neural networks may comprise one or more layers, each layer consisting of a multitude of neurons. Each of the neural networks may comprise an activation function.

In examples, each of the neural network may be trained on the above defined dataset obtained at S152, consisting of randomly generated instances of the predetermined parameters and the determined instances of the predetermined characterization of hemodynamic localization noise. In examples, training each of the neural networks may comprise an optimization loss function. The loss function may be a mean square error (MSE), given, e.g., by the formula: $L \left(θ\right) = {MSE}_{p} \left({C}_{mn}^{loc - i}\left(p\right),{NN}_{θ}\left(p\right)\right)$ where the index i indicates an artery, the quantity ${C}_{mn}^{loc - i} \left(p\right)$ is the above defined covariance matrix *C^{loc}*(*t,t'*|*p*) of the artery *i, NN_{θ}* is one of the neural network to be trained on the above defined dataset obtained at S152.

The obtaining S100 comprises obtaining S103 a pre-trained measurement-parameter machine-learning function having been trained for outputting an instance of the predetermined parameters from measurements of the hemodynamic vascular behavior provided as input.

The measurements of the hemodynamic vascular behavior comprise instances of hemodynamic signals characterizing the blood flow of a predetermined subset of arteries of the plurality of arteries of the patient. Each of the hemodynamic signals characterizing the blood flow of a predetermined subset of arteries of the plurality of arteries of the patient may be comprised in the predetermined hemodynamic signals. In other words, the measurements of the hemodynamic vascular behavior may be an instance of a subset of the predetermined hemodynamic signals. In particular, each of the hemodynamic signals may be, e.g., blood pressure, blood flow rate, artery cross-sectional area, or blood velocity of a respective artery (e.g., at the middle point of the respective artery).

The pre-trained measurement-parameter machine-learning function obtained at S103 may comprise a pre-trained measurement-parameter neural network model for one or more (e.g., each) of the parameters characterizing a respective artery of the plurality of arteries of the patient comprised in the predetermined parameters. Each of said pre-trained measurement-parameter neural network models outputting instances of the parameter characterizing the respective artery from measurements of the hemodynamic vascular behavior obtained as input. That is, the pre-trained measurement-parameter machine-learning function obtained at S103 may comprise one or more (e.g., one for each parameter comprised in the predetermined parameters) pre-trained measurement-parameter neural network models, each of the pre-trained measurement-parameter neural network models outputting an instance of a respective parameter comprised in the predetermined parameters from measurements of the hemodynamic vascular behavior obtained as input. Having a different pre-trained measurement-parameter neural network for one or more (e.g., each) of the parameter may be advantageous as each of the pre-trained measurement-parameter neural networks may be faster and more efficient compared to a model trained to output all the parameters altogether.

With reference to the flowchart of FIG. 2, the obtaining S103 of the pre-trained measurement-parameter machine-learning function may comprise obtaining S153 a measurement-parameter dataset. The measurement-parameter dataset may comprise entries, each entry comprising measurements of the hemodynamic vascular behavior and an instance of the predetermined parameters.

The obtaining S153 of a measurements-parameter dataset may comprise generating the dataset with data of virtual patients. Generating a dataset with data of virtual patients may be beneficial, as the generated dataset may be tailored for training the required machine-learning function.

The obtaining S153 may comprise, for each given entry of the parameter-signal dataset obtained at S151, obtaining measurements of the hemodynamic vascular behavior from the instances of the hemodynamic signals characterizing the blood flow of the predetermined subset of arteries comprised in the given entry. The obtaining of the measurements of the hemodynamic vascular behavior may comprise restricting the instance of the predetermined hemodynamic signals (comprised in the given entry) to the hemodynamic signals characterizing the blood flow of the predetermined subset of arteries of the plurality of arteries of the patient.

The obtaining S153 may further comprise obtaining noisy measurements of the hemodynamic vascular behavior by adding to the obtained measurements of the hemodynamic vascular (e.g., the restriction of the instance of the predetermined hemodynamic parameters) a noise. In specific, the obtaining S153 may comprise obtaining one or more noisy measurements of the hemodynamic vascular behavior by adding to the obtained measurements of the hemodynamic vascular respective instances of a noise. Therefore, the obtaining S153 may comprise determining one or more entries of the measurement-parameter dataset from each given entry of the parameter-signal dataset, each of which obtained by adding a respective instance of a noise to a restriction of the instance of the predetermined hemodynamic signals comprised in the given entry.

In examples, adding to the obtained measurements of the hemodynamic vascular behavior a noise may comprise adding at each given instance of a respective hemodynamic signal comprised in the measurements of the hemodynamic vascular behavior a noise relative to the given instance. The noise relative to different given instances may be independent. In other words, adding to the obtained measurements of the hemodynamic vascular behavior a noise may comprise adding an independent noise to each of the instances of the hemodynamic signal comprised in the measurements of the hemodynamic vascular behavior.

The medical measuring device used at S110 may not achieve absolute precision and may be subject to inherent measurement inaccuracies. These inaccuracies arise from various sources, including electronic interference, environmental conditions, and the intrinsic limitations of the sensor components. As a result, the measurements obtained are influenced by random noise, which introduces variability and uncertainty into the recorded data. This noise is characterized by its variance, denoted as ${σ}_{mes}^{2}$*,* which quantifies the degree of dispersion around the true value being measured. The presence of noise with variance ${σ}_{mes}^{2}$*,* affects the overall reliability and accuracy of the measurements. Therefore, it may be beneficial in the method to consider this noise while obtaining S153 the measurements-parameter dataset. Indeed, the measurement-parameter machine-learning function that may be trained with the dataset obtained at S153 may take an input measurements of the hemodynamical vascular behavior of the patient obtained with the medical measuring device (e.g., at S110).

Therefore, in examples, the method may comprise obtaining the variance ${σ}_{mes}^{2}$ characterizing a reliability of the medical measuring device used at S110. The variance may be obtained from the instructions of the medical measuring device, or by analyzing the interval of confidence of the data obtained with the medical measuring device. In examples, the noise to be added in each of the instances of the hemodynamic signal comprised in the measurements of the hemodynamic vascular behavior may depend on the variance ${σ}_{mes}^{2}$*.* For example, in examples, the obtaining S153 may comprise adding to each of the instances of hemodynamic signals comprised in the obtained measurements of the hemodynamic vascular behavior an instance of a noise defined by the covariance $δ \left(t-t′\right) {σ}_{mes}^{2}$, where ${σ}_{mes}^{2}$ is the variance characterizing a reliability of the medical measuring device used at S110 and *δ* is the delta function (i.e., the distribution for which *∫ f*(*t*)*δ*(*t - t'*)*dt* = *f*(*t'*) for every function *f*).

In examples, the noise may further be characterized by the covariance matrix obtained at S152, e.g., *C^{loc}*(*t,t'*|*p*), where p is the instance of the predetermined parameters of the given entry of the parameter-signal (that is, the instance of the predetermined parameters associated with the measurements of the vascular behavior of the patient). Therefore, in examples, the obtaining S153 may comprise adding to each the instances of hemodynamic signals comprised in the obtained measurements of the hemodynamic vascular behavior of the patient an instance of a noise defined by the covariance given by: ${C}^{mes} \left(t,t′\right) = δ \left(t-t′\right) {σ}_{mes}^{2} + {C}^{loc} \left(t,\left.t′\right|p\right) .$

In examples, adding an instance of a noise (e.g., defined by any of the above defined covariances) may comprise discretizing the signal over the time steps, as is well known in the art.

The obtaining S153 may further comprise solving an inverse problem, thereby obtaining an instance of the predetermined parameters that are associated with the noisy measurements of the hemodynamic vascular behavior. That is, for each of the obtained noisy measurements of the vascular behavior, the obtaining S153 may comprise determining an instance of the predetermined parameters that are associated with the noisy measurements of the vascular behavior. By associated with it is meant, e.g., that the instances of the hemodynamic signals comprised in the noisy measurements of the vascular behavior are (e.g., exactly, or approximate) the instances of the hemodynamic signals obtained by using the one-dimensional blood flow modeling solver (e.g., the above defined solver) from the instance of the predetermined parameters.

In formulae, the obtaining S153 may comprise determining a function given by: $I : O ↦ \left(O\right) = {p}^{MAP}$ where *O* is a measurement of the vascular behavior and *p^{MAP}* is the associated instance of the predetermined parameters.

Solving the inverse problem may comprise using the Bayes method. Using the Bayesian method to solve inverse problems is advantageous because it provides a systematic probabilistic framework that integrates prior knowledge with the noisy data. This approach effectively quantifies and manages uncertainty, making solutions more reliable. By treating unknown parameters as random variables and updating their distributions using Bayes theorem, the Bayesian method enables more accurate and informed inferences.

Using the Bayes method may comprise considering that the instance of the predetermined parameters *p* to be determined is a random variable. Bayes inference may be stated in formulae as: ${π}_{post} \left(\left.p\right|O\right) = \frac{{π}_{l} \left(\left.O\right|p\right) {π}_{prior} \left(p\right)}{{\int }_{p ′ ∈ {ε}_{p}} {π}_{l} \left(\left.O\right|p′\right) {π}_{prior} \left(p′\right) dp ′} ∝ {π}_{l} \left(\left.O\right|p\right) {π}_{prior} \left(p\right)$ where *πₚₒₛₜ*(*p*|*O*) is the density of the instances of the predetermined parameters, *πₗ*(*O*|*p*) is the likelihood function, and *πₚᵣᵢₒᵣ*(*p*) is the prior density of the parameters p.

The prior density of the predetermined parameters may be a constant function or may reflect the probability that the instance *p* of the predetermined parameters is a possible instance of an actual patient (e.g., a human). In examples, some of the combinations of instances of parameters may be improbable (e.g., some arteries very long with respect to a respective mean, and close by arteries very short with respect to a respective mean) and the prior density may characterize this phenomenon. Therefore, the method may comprise obtaining a prior density to be used in the obtaining S153.

In examples, the measurement noise may be mutually independent from the parameters, and the noise distribution may be denoted *π_{ε}*. In examples, the noise model may not involve any hyperparameters, and the likelihood function may be expressed with the following formula: ${π}_{l} \left(\left.O\right|p\right) = {π}_{ϵ} \left(O-M\left(p\right)\right)$ where *M* is the measurement operator, that is, the operator that associates to each instance of the predetermined parameters measurements of the vascular behavior by using the one-dimensional blood flow modeling solver (e.g., the above defined solver).

In examples, it may be beneficial to use the pre-trained parameter-signal machine-learning function obtained at S101 to approximate the operator *M*, instead of using the one-dimensional blood flow modeling solver. Indeed, to solve the Bayes inverse problem it may be necessary to compute the measurement operator *M* on a multitude of instances of the predetermined parameters. As using the one-dimensional blood flow modeling solver may take a long time, and the pre-trained parameter-signal machine-learning function obtained at S101 is reliable (as proved during implementations and shown, e.g., in FIG. 3), it may be beneficial to use a measurement operator *M^{NN}* derived from the pre-trained parameter-signal machine-learning function. In specific, *M^{NN}*(*p*), where *p* is an instance of the predetermined parameters, is a measurements of the hemodynamic vascular behavior consisting of a restriction (as explained above) of an instance of the predetermined hemodynamic signals obtained as output of the parameter-signal machine-learning function obtained at S101. In examples, the pre-trained parameter-signal machine-learning function may comprise a pre-trained parameter-signal neural network for each hemodynamic signal comprised in the predetermined hemodynamic signals, so that ${M}^{NN} \left(p\right) = {\left({M}_{i}^{NN}\left(p\right)\right)}_{i}$, where *i* ranges over all hemodynamic signal comprised in the hemodynamic signals characterizing the blood flow of a predetermined subset of arteries of the plurality of arteries of the patient.

In examples, obtaining an instance of the predetermined parameters that are associated with the noisy measurements of the hemodynamic vascular behavior may comprise solving a non-linear optimization problem by maximizing the log value of the posterior density, or by minimizing the negative log-posterior, in formulae: ${p}^{MAP} = {argmin}_{p ∈ {E}_{p}} \left(-{\sum }_{i = 1}^{{N}_{O}} \left(\begin{matrix}- \frac{{N}_{T}}{2} log \left(2π\right) - \frac{1}{2} log \left(\left|Σ\right|\right) \\ - \frac{1}{2} {\left({O}_{i}-{M}_{i}^{NN}\left(p\right)\right)}^{T} {Σ}^{- 1} \left({O}_{i}-{M}_{i}^{NN}\left(p\right)\right)\end{matrix}\right) - log \left({π}_{prior}\left(p\right)\right)\right)$ where:
o *p^{MAP}* is the instance of the predetermined parameters that are associated with the noisy measurements and that are to be added in the measurement-parameter dataset obtained at S153,
∘ ε*ₚ* is a space of all the possible instances of the predetermined parameters,
∘ *p* is an instance of the predetermined parameters,
∘ *N_{O}* is a number of hemodynamic signals characterizing the blood flow of a predetermined subset of arteries of the plurality of arteries of the patient,
∘ *N_{T}* is a number of time steps of each of the hemodynamic signals (e.g., the number of time steps that may be used to discretize the time variable),
∘ *Σ* is a covariance matrix characterizing the noise,
∘ *O* is the noisy measurements of the hemodynamic vascular behavior that is decomposed as *O* = (*Oᵢ*)*ᵢ*, where the index *i* ranges over all hemodynamic signal comprised in the hemodynamic signals characterizing the blood flow of a predetermined subset of arteries of the plurality of arteries of the patient, that is, *Oᵢ* is an instance of a *i*-th hemodynamic signal comprised in the noisy measurements of the hemodynamic vascular behavior,
∘ *M^{NN}*(*p*) is a measurements of the hemodynamic vascular behavior consisting of a restriction of an output of the parameter-signal machine-learning function obtained at S101 and ${M}^{NN} \left(p\right) = {\left({M}_{i}^{NN}\left(p\right)\right)}_{i}$ as explained above, that is, ${M}_{i}^{NN} \left(p\right)$ is an instance of the i-th hemodynamic signal of the measurements of the hemodynamic vascular behavior obtained from the instance p of the predetermined parameters, and
∘ *πₚᵣᵢₒᵣ*(*p*) is the priority density of the parameters.

In examples, the obtaining S153 may comprise determining entries of the measurement-parameter dataset, each entry comprising a noisy measurement of the vascular behavior and the associated *p^{MAP}*, e.g., as obtained with the above formula.

The obtaining S103 of the pre-trained measurement-parameter machine-learning function may further comprise training S163 a machine-learning function on a measurement-parameter dataset, thereby obtaining the pre-trained measurement-parameter machine-learning function. The measurement-parameter dataset may be the dataset obtained at S153 and may be obtained according to the above example.

In examples, the machine-learning function to be trained at S163 may comprise, for one or more of (e.g., for each) of the parameters comprised in the predetermined parameters, a neural network. Therefore, in examples, the method may not use any correlation between the different parameters. Each of the neural networks may comprise one or more layers (e.g., 4 layers), each layer consisting of a multitude of neurons (e.g., 50 neurons). Each of the neural networks may comprise an activation function (e.g., a Tanh function) and may have a final linear layer.

In examples, each of the neural networks may be trained on the above defined dataset obtained at S153 consisting of noisy measurements of vascular behavior and instances of predetermined parameters found with the Bayes method. In examples, the measurement-parameter machine-learning function may be denoted by Î*_{θ}*, where *θ* contains all the neural network parameters. The measurement-parameter machine-learning function may be defined by the following formula: ${\hat{I}}_{θ *} : O ↦ {\hat{I}}_{θ *} \left(O\right) = \left.\hat{I}\left(O,θ*\right)\right| θ * = {argmin}_{θ} {L}_{p} \left(θ\right)$ where *O* are measurements of the hemodynamic vascular behavior. In examples, training each of the neural networks may comprise an optimization loss function 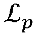 (*θ*) given by ${L}_{p} \left(θ\right) = {E}_{p} \left[{\left‖\hat{I}\left(O, θ\right)-p\right‖}_{{ε}_{p}}\right] = {\int }_{{ε}_{p}} {\left‖\hat{I}\left(O, θ\right)-p\right‖}_{{ε}_{p}} {ℙ}_{p}$ ${\hat{L}}_{p} \left(θ\right) = \frac{1}{N} {\sum }_{i = 1}^{N} {\left‖\hat{I}\left({O}_{i}, θ\right)-{p}_{i}\right‖}_{{E}_{p}} {\to }_{N - ∞} {L}_{p} \left(θ\right)$

In examples, each of the neural networks may be trained according to the following:

| GPU | Architecture | Activation function | Optimizer | Learning rate | Number of epochs | Training time |
|---|---|---|---|---|---|---|
| NVIDIA A6000 | 4 layers of *Nₗ* - 50 neurons | Tanh with final linear layer | Adam | 1e-5 | ~ 15K | ~ 300 s |

The method further comprises obtaining S110 the measurements of the hemodynamical vascular behavior of the patient with a medical measuring device. The obtaining S110 may be effectuated by, e.g., a health specialist, or any user capable to use the medical measuring device. Alternatively or additionally, the obtaining S110 may be from a patient database, e.g., upon an input of a patient identifier (ID) by a health specialist. In examples, the patient ID may comprise one or more of a last name, a first name, a registration ID at a clinic/hospital, an insurance number and/or a date of birth. Said patient database may be a local database stored on a local hard drive, or a database stored on a cloud.

The obtained measurements of the hemodynamic vascular behavior of the patient with the medical measure device comprise patient-dependent measured instances of the hemodynamic signal, one for each hemodynamic signals characterizing the blood flow of a predetermined subset of arteries of the plurality of arteries of the patient. As stated above, they may comprise, e.g., blood pressure, blood flow rate, blood axial velocity, vessel cross-sectional area of a combination thereof of the predetermined subset of arteries. In examples, the measurements may be obtained by different non-intrusive techniques, e.g.,
- blood pressure: Applanation tonometry and volume-clam method;
- Blood velocity: Phase contrast MRI and pulsed doppler ultrasound;
- Vessel cross-sectional area: Magnetic resonance angiography and B-mode ultrasonography; and
- Blood flow rate: the flow rate may be measured by combining velocity and cross-sectional area measurements.

The method further comprises generating S120 a plurality of noisy measurements of the hemodynamic vascular behavior of the patient. As already stated in length above, the medical measuring device used at S110 may measure the hemodynamic signal at a section of the relative artery, however the precise position of the section may be not be determined (e.g., the health specialist may not be able to find the exact middle point of an artery). Therefore, the measurements of the vascular behavior of the patient obtained at S110 may comprise a noise/an error due to the localization of the artery where the measurements are effectuated (e.g., the method may expect a measurement at a middle point, but the measurement may not be exactly at the middle point). The generating S120 gives therefore an interval of confidence of the measurements with respect to the localization error.

Moreover, the medical measuring device used at S110 may not achieve absolute precision and may be subject to inherent measurement inaccuracies (e.g., there may be some error due to the medical measuring device itself). The method may therefore, optionally, consider the inherent noise of the medical measuring device at S120.

The generating S120 comprises providing S121 as input the obtained measurements of the hemodynamic vascular behavior of the patient (obtained at S110) to the pre-trained measurement-parameter machine-learning function (obtained at S103, optionally trained at S163, e.g., the one of the above example), thereby obtaining (as an output) a patient-dependent instance of the predetermined parameters. By providing as input, it is meant that the pre-trained measurement-parameter machine-learning function is applied at the input (that is at the measurements of the hemodynamic vascular behavior of the patient). The output of the pre-trained measurement-parameter machine-learning function is an instance of the predetermined parameter that depends on the actual data of the patient, that is, a patient-dependent instance of the predetermined parameters. Therefore, the providing S121 comprises obtaining a patient-dependent instance of the predetermined parameters.

The generating S120 comprises providing S122 as input the obtained patient-dependent instance of the predetermined parameters (obtained at S121) to the pre-trained parameter-noise machine-learning function (obtained at S102, optionally trained at S162, e.g., the one of the above example), thereby obtaining a patient-dependent instance of the predetermined characterizations of hemodynamic localization noise. By providing as input, it is meant that the pre-trained parameter-noise machine-learning function is applied at the input (that is at patient-dependent instance of the predetermined parameters). The output of the pre-trained parameter-noise machine-learning function is an instance of the predetermined characterizations of hemodynamic localization noise that depends on the actual data of the patient, that is, a patient-dependent instance of the predetermined characterizations of hemodynamic localization noise. Therefore, the providing S121 comprises obtaining a patient-dependent instance of the predetermined characterizations of hemodynamic localization noise.

The generating S120 comprises determining S123 a patient-dependent noise generator from the patient-dependent instance of the predetermined characterizations of hemodynamic localization noise (obtained at S122). The patient-dependent noise generator is able to modify the measurements of the hemodynamic vascular behavior of the patient (obtained at S110) by adding noise. The noise quantifies the possible errors in the measurements of the hemodynamic vascular behavior of the patient with the medical measuring device.

The patient-dependent noise generator may comprise one or more noise distributions, e.g., each defined with a covariance. For example, the patient-dependent noise generator may comprise a noise distribution defined by a covariance for each of the hemodynamic signals comprised in the hemodynamic signals characterizing the blood flow of a predetermined subset of arteries of the plurality of arteries of the patient. The generating S120 may further comprise adding to the measurements of the hemodynamic vascular behavior of the patient (obtained at S110) instances of the noise distributions, thereby obtaining a plurality of noisy measurements of the hemodynamic vascular behavior of the patient (one for each instance of the noise distributions).

Therefore, the generating S120 may generate a plurality of noisy measurements of the hemodynamic vascular behavior of the patient. In specific, each of the noisy measurements of the hemodynamic vascular behavior of the patient generated at S120 may be generated by adding to each of the patient-dependent measured instances of the hemodynamic signal an instance of the noise generated with the noise generator. In examples, each characterization of noise comprised in the predetermined characterizations of hemodynamic localization noise may be a covariance matrix, and the patient-dependent noise generator may comprise one or more multivariate normal distributions, each of the multivariate normal distribution determined by an instance of a covariance matrix comprised in the patient-dependent instance of the predetermined characterizations of hemodynamic localization noise.

In examples, the output of the pre-trained parameter-noise machine-learning function (obtained at S102) may comprise, for each hemodynamic signal characterizing the blood flow of a predetermined subset of arteries of the plurality of arteries of the patient, a covariance matrix *C^{loc}*(*t,t'*|*p*) characterizing a localization noise. Therefore, in examples, the noise generator may comprise the multivariate normal distributions determined by the above covariance matrices.

In examples, the generating S120 may further comprise obtaining a noise confidence level of the measurements of the hemodynamical vascular behavior of the patient with the medical measuring device. The patient-dependent noise generator determined at S120 may further be determined from the obtained noise confidence level. In examples, the noise confidence level may be given by a variance ${σ}_{mes}^{2}$ depending on the medical measuring device, as explained above. Therefore, the generating S120 may comprise obtaining the variance ${σ}_{mes}^{2}$*.*

In examples, the patient-dependent noise generator determined at S120 may be determined by covariance given by: ${C}^{mes} \left(t,t′\right) = δ \left(t-t′\right) {σ}_{mes}^{2} + {C}^{loc} \left(t,\left.t′\right|p\right) .$

For example, it may be the multivariate normal distribution with the covariance given above. Each of the plurality of noisy measurements of the hemodynamic vascular behavior of the patient generated at S120 may therefore be written as ${S}^{mes} = S \left(\left.{x}_{c}\right|p\right) + \left[U\right] {\left[Λ\right]}^{\frac{1}{2}} g = \left(\begin{matrix}{S}^{mes} \left({t}_{1}\right) \\ ⋮ \\ {S}^{mes} \left({t}_{{N}_{t}}\right)\end{matrix}\right) , g ∼ N \left(0, {I}_{{N}_{t}}\right)$ $\left[{C}^{mes}\right] = \left[{C}^{mes}\left({t}_{i}, {t}_{j}\right), i,j∈\left〚1, {N}_{t}\right〛\right] = \left[U\right] \left[Λ\right] {\left[U\right]}^{T}$ $\left[U\right] {\left[U\right]}^{T} = {\left[U\right]}^{T} \left[U\right] = \left[{I}_{{N}_{t}}\right]$ where *U* is an orthogonal matrix used for an orthogonal diagonalization of *C^{mes},* A is a diagonal matrix, *Nₜ* is the size of the discretization in the time variable, *x_{c}* is the location of the center of the respective artery, *S*(*x_{c}*|*p*) is the patient-dependent measured instance of a hemodynamic signal, and *S^{mes}* is the patient-dependent noisy instance of the hemodynamic signal.

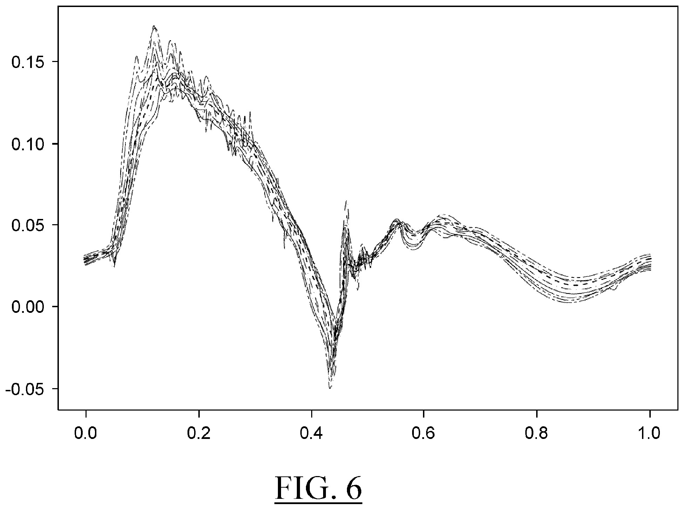

FIG. 6 shows an example of a plurality of noisy measurements of the blood flow velocity at the central point of the subclavian artery that may be generated according to S120.

In examples, therefore, predicting the associated instance of the predetermined parameter for a given noisy measurement of the vascular behavior of the patient may be obtained in real time (e.g., after less than a second) using the pre-trained measurement-parameter machine-learning function (obtained at S103, optionally trained at S163). This, therefore, may yield a multitude of instance of the predetermined parameters, which may correspond to a noise level of the instance of the predetermined parameters associated with the measurements of the hemodynamic vascular behavior of the patient obtained at S110. The patient-dependent noise generator may be personalized to the identified Maximum-A-Posteriori (MAP) parameter using the formula: ${S}_{i} \left({p}^{MAP - NN}\right) = {F}_{i}^{NN} \left({p}^{MAP - NN}\right) + {ϵ}_{i}^{NN} \left({p}^{MAP - NN}, {σ}_{mes}^{2}\right) .$

The method further comprises providing S130 as input each of the noisy measurements of the hemodynamical vascular behavior of the patient (obtained at S120) to the pre-trained measurement-parameter machine-learning function (obtained at S103, optionally trained at S162, e.g., the one of the above example), thereby obtaining a plurality of noisy patient-dependent instances of the predetermined parameters. By providing as input, it is meant that the pre-trained measurement-parameter machine-learning function is applied at the input (that is at each of the noisy measurements of the hemodynamical vascular behavior of the patient). Each of the outputs of the pre-trained measurement-parameter machine-learning function is an instance of the predetermined parameter that depends on the actual data of the patient and on the patient-dependent noise generator (determined at S123), that is, a noisy patient-dependent instance of the predetermined parameters. Therefore, the providing S120 comprises obtaining a plurality of noisy patient-dependent instances of the predetermined parameters.

The method further comprises providing S140 as input each of the obtained plurality of noisy patient-dependent instances of the predetermined parameters (obtained at S130) to the pre-trained parameter-signal machine-learning function, thereby obtaining a plurality of noisy patient-dependent instances of the predetermined hemodynamic signals. By providing as input, it is meant that the pre-trained parameter-signal machine-learning function is applied at the input (that is at each of the noisy patient-dependent instances of the predetermined parameters). Each of the outputs of the pre-trained parameter-signal machine-learning function is an instance of the predetermined hemodynamic signals that depends on the actual data of the patient and on the patient-dependent noise generator (determined at S123), that is, a noisy patient-dependent instances of the predetermined hemodynamic signals. Therefore, the providing S140 comprises obtaining a plurality noisy patient-dependent instances of the predetermined hemodynamic signals.

The plurality of noisy patient-dependent instances of the predetermined hemodynamic signals obtained at S140 corresponds to an uncertainty of the prediction, the uncertainty depending on the uncertainty of the measurements obtained at S110. That is, the measurements obtained at S110 may not be the exact measurements, as explained in length above. The plurality of noisy measurements generated at S120 quantifies the uncertainty of the measurement obtained at S110. This plurality of measurements, that is, this quantified uncertainty, is then further used in the method (throughout S130 and S140) to obtain the plurality of noisy patient-dependent instances of the predetermined hemodynamic signals. The plurality of the noisy patient-dependent instances of the predetermined hemodynamic signals comprises the actual (i.e., real) instance of the predetermined hemodynamic signals of the patient. Therefore, the plurality quantifies the uncertainty of the obtained prediction of the hemodynamic vascular behavior of the patient. The method may further comprise deriving statistical information from the plurality noisy patient-dependent instances of the predetermined hemodynamic signals obtained at S140 (e.g., mean or variance). The method may comprise outputting the signal and/or the statistical information to the user. The output may comprise a graph for each hemodynamic signal comprised in the predetermined hemodynamic signals, the graph showing the confidence level at each point in time.

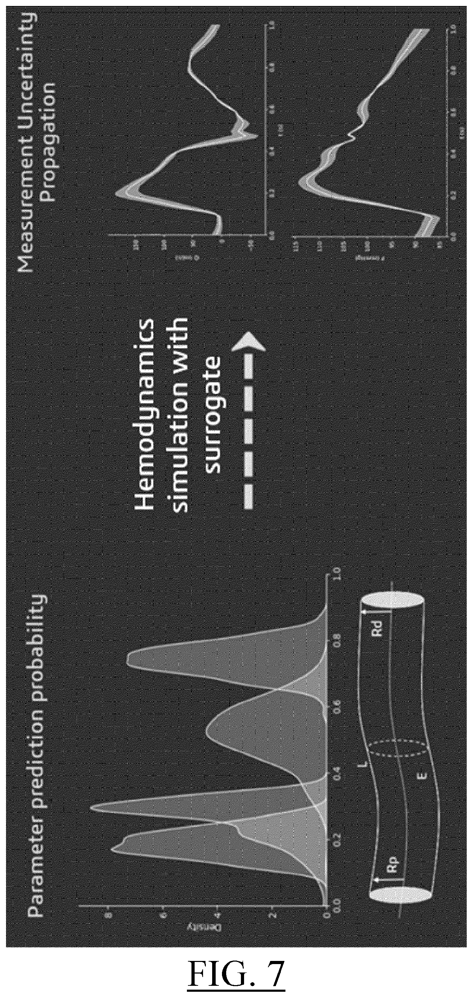

FIG. 7 illustrates how in an implementation of the method, the parameter prediction probabilities may give graphs for hemodynamic signals showing the confidence level at each point in time. The confidence level may be express with a band of variable width.

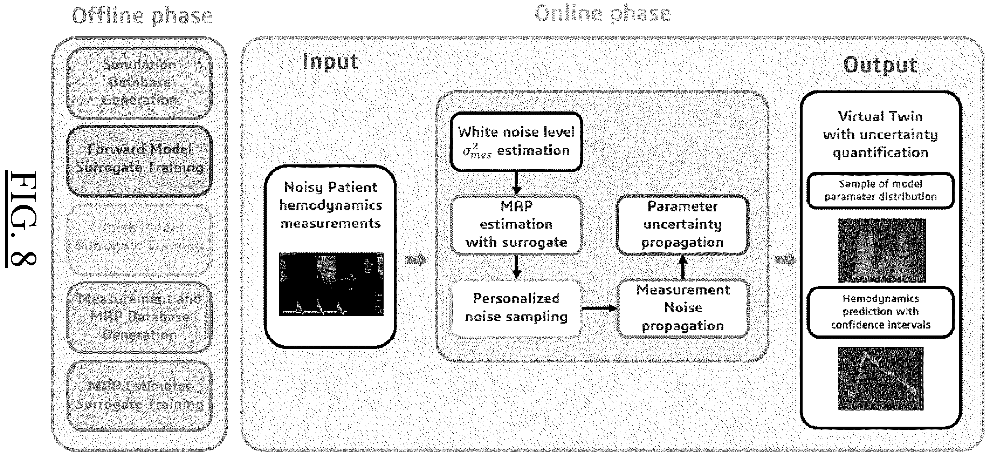

FIG. 8 illustrates a pipeline of an example of an implementation of the method. The example of the implementation comprises an offline phase and an online phase. The offline phase may comprise obtaining one or more datasets and training one or more machine-learning functions, such as S100, S151, S152, S153, S161, S162, and S163. The online phase may comprise obtaining S110 measurements of the hemodynamical vascular behavior of a patient and steps S120, S130, and S140. The example of the implementation may further comprise displaying the obtained results on a Virtual Twin, the results comprising the uncertainty quantification.

The method, comprising the customizing of a digital twin of a patient (that may have been virtual) was implemented, and such implementation is described with reference to FIGs. 9-13. FIG. 9 shows a vascular model of the digital twin comprised and used in the implementation. The vascular model comprises a plurality of arteries and the heart of the patient.

The implemented vascular model of the digital twin was used in the implementation to predict the amount of blood flowing into the brain of the patient starting from (non-invasive) measurements obtained at S110 (e.g., ultrasound measurements). In the implementation, a health specialist (e.g., a cardiologist) used the digital twin of a patient to predict whether the failure of the heart of the patient may lead to an insufficient perfusion in oxygen supply to the brain. In the implementation, the obtaining S110 comprised, by the health specialist, obtaining non-intrusive ultrasound measurements. The ultrasound measurements are used by the pre-trained machine-learning function (obtained at S100) to simulate the hemodynamic flow of the digital twin in real time. As already described in length above, fitting the simulation to all the ultrasounds is a complex task that requires tuning more than 300 parameters. The trained machine-learning functions (obtained at S100) give an automatic personalization of all the parameters of all the arteries and of all the hemodynamic signals.

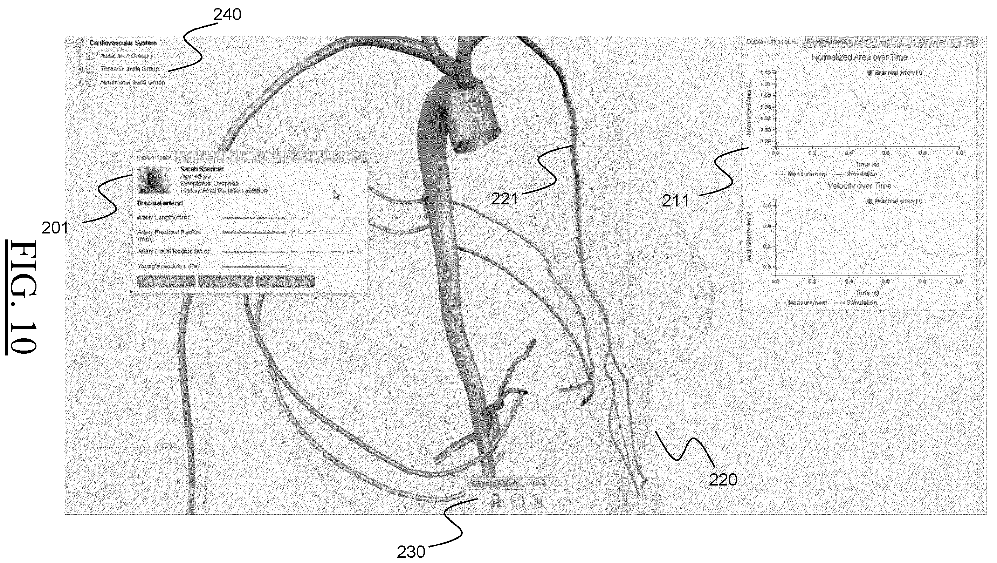

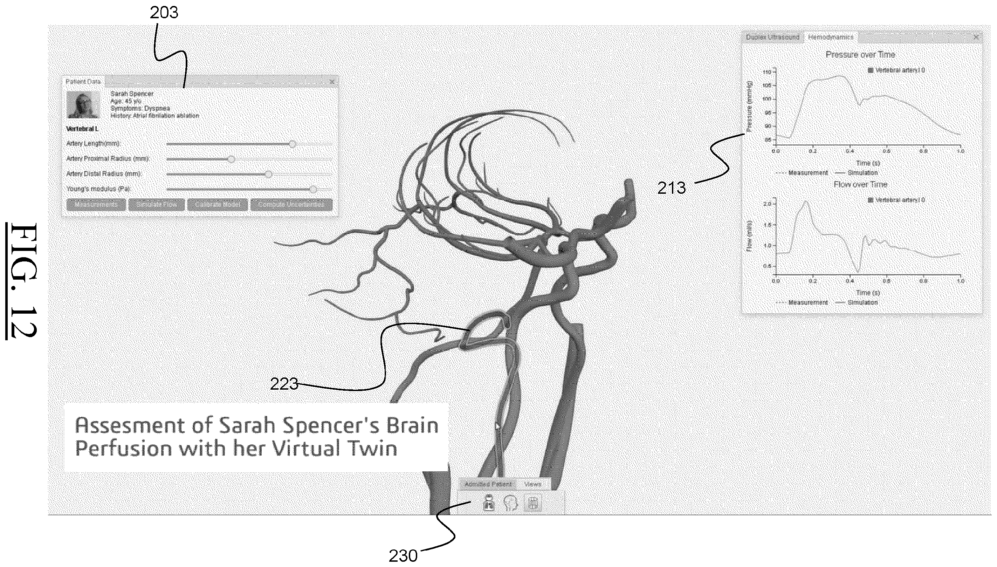

FIGs. 10-11 show a GUI (Graphical User Interface) used in the implementation of the method and FIGs. 12-13 show a simplified GUI that was also used in the implementation of the method. In implementations, the method may comprise a graphical interface similar to the one of FIGs. 10-11 and/or FIGs. 12-13. The GUI of FIGs. 10-11 comprises a feature tree 240 that may be used to navigate the digital twin, e.g., to choose an artery of the plurality of arteries of the patient comprised in the vascular model of the digital twin. The GUI of FIGs. 10-11 shows a representation of the digital twin 220, the GUI of FIGs. 12-13 shows a simplified representation only showing some of the arteries of the plurality of arteries of the patient. The GUI of FIGs. 10-11 and the GUI of FIGs. 12-13 both comprise a window (201 to 204) comprising the personal information of the patient comprising, name, age, symptoms, and medical history. In each of FIGs. 10-13 at least one artery is selected (221 to 224) and the parameters associated to the at least one artery are shown in the window 201 to 204 of the respective figure. The parameters of the window 201 to 204 (comprised in the predetermined parameters) comprise artery length, artery proximal radius, artery distal radius, and Young's modulus. The GUI of FIGs. 10-11 and the GUI of FIGs. 12-13 both comprise a signal-window 211-4 showing the hemodynamic signals of the selected arteries. The hemodynamic signals comprised in the signal-window 211-4 are the normalized area over time and velocity over time. The GUI of FIGs. 10-11 and the GUI of FIGs. 12-13 both comprise a menu 230 that may be used to navigate the digital twin.

FIG. 10 shows the digital twin where the brachial artery 221 in the representation of the digital twin 220 is selected. The selection may have been, e.g., with a mouse and implementations of the method may comprise any way of interacting with the digital twin (e.g., with a mouse or a touchpad). The parameters of the brachial artery are shown in the window 201. As it may be seen, these parameters are not calibrated, they are all equal to a constant value. The signal-window 211 comprises two hemodynamic signals of the brachial artery 221 that were obtained from the patient with a medical measuring device (at S110, hence comprised in the measurements of the hemodynamic behavior of the patient). The signals shown in the signal-window 211 comprise the normalized area over time and velocity over time. The measurements may have been obtained by the health specialist with ultrasound measurements.

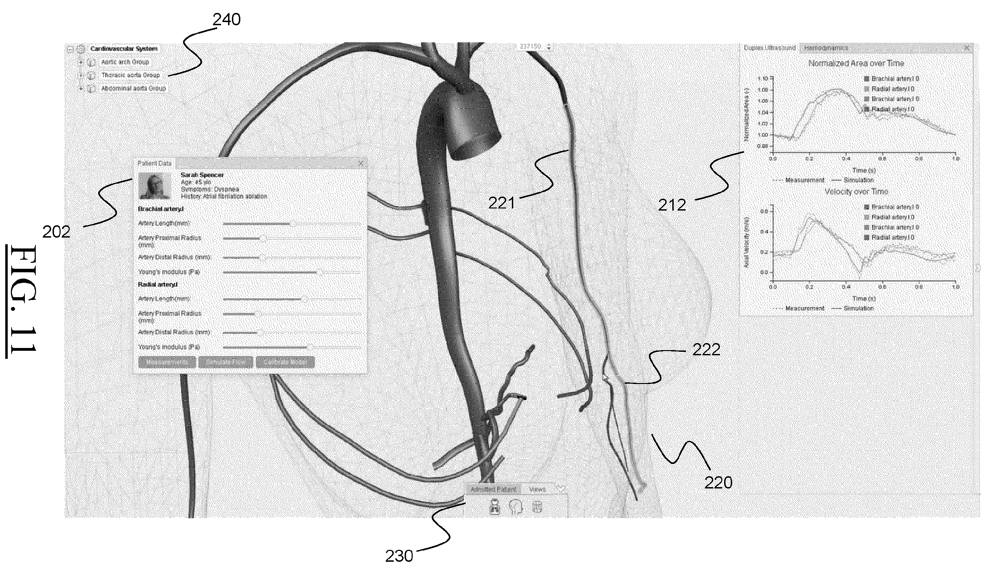

FIG. 11 show the digital twin where the brachial artery 221 and the radial artery 222 are selected. As it may be seen in the window 202, the parameters have been customized to the patient starting from the hemodynamic signals that are shown in the signal-window 212. The customization has been determined using the one or more pre-trained machine-learning functions obtained at S100 and, specifically, the pre-trained measurement-parameter machine-learning function obtained at S103. In particular, the signal-window 212 shows the measured hemodynamic signals for both the brachial artery 221 and the radial artery 222. The hemodynamic signals were obtained according to the implementation of the method (e.g., with the pre-trained parameter-signal machine-learning function obtained at S101). The generated hemodynamic signals are very close to the measured hemodynamic signals. This is a further proof of the efficacy of the method.

In FIG. 11, the window 202 comprises an interactive element called "Calibrate Model" that may be used in the implementation to calibrate the parameter starting from measurements of the vascular behavior of the patient (e.g., with the pre-trained machine-learning function obtained at S103). The window 202 further shows an interactive element called "Simulate Flow" that may be used to, starting from the calibrated parameters, predict the hemodynamic signals of all the arteries comprised in the virtual twin (i.e., obtain an instance of the predetermined hemodynamic signal, such an instance may be obtained, e.g., with the pre-trained parameter-signal machine-learning function obtained at S101).

FIG. 12 shows the arteries of the brain of the digital twin, where the vertebral artery 223 is selected. The window 203 shows the customized parameters of the artery and the signal-window 213 shows the predicted hemodynamic signals. The health specialist may, using the hemodynamic signals shown in the signal-window 213, assess the patient's blood perfusion in the brain arteries to check if the brain is impacted by the patient's heart failure.

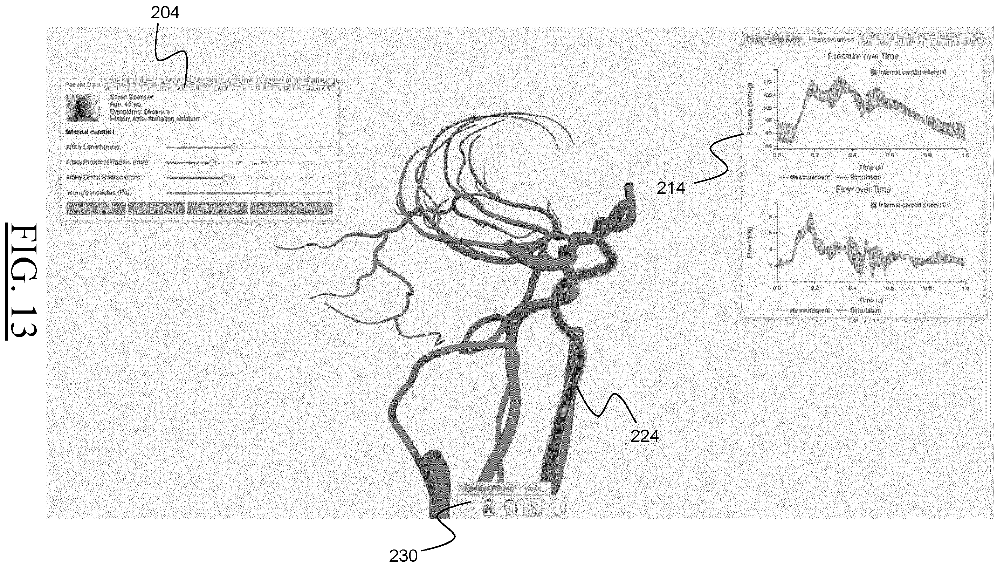

However, the health specialist confronted with FIG. 12 and in particular with the signal-window 213, cannot judge how reliable the hemodynamic signal of the signal-window 213 is. Indeed, as already stated above, there are multiple sources of uncertainties in personalizing a digital twin, such as measurement noise. Thanks to the method, the health specialist may now obtain the hemodynamic signals with a confidence interval, as shown in FIG. 13.

Indeed, FIG. 13 shows the arteries of the brain of the digital twin, where the internal carotid artery 224 is selected. The window 204 shows the customized parameters of the artery and the signal-window 214 shows the predicted hemodynamic signals. The signal-window 214 also shows the quantified uncertainties, through a confidence bar around the predicted hemodynamic signal. Thanks to these quantified uncertainties, the health specialist could estimate the confidence they had on the predicted perfusion values in the brain arteries, and they could assess whether in the worst prediction the blood supply to the brain was sufficient.

In FIG. 13, the window 204 comprises an interactive element called "Compute Uncertainties" that may be used in the implementation to compute the uncertainties according to the method.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g., one for the program, and possibly one for the database).

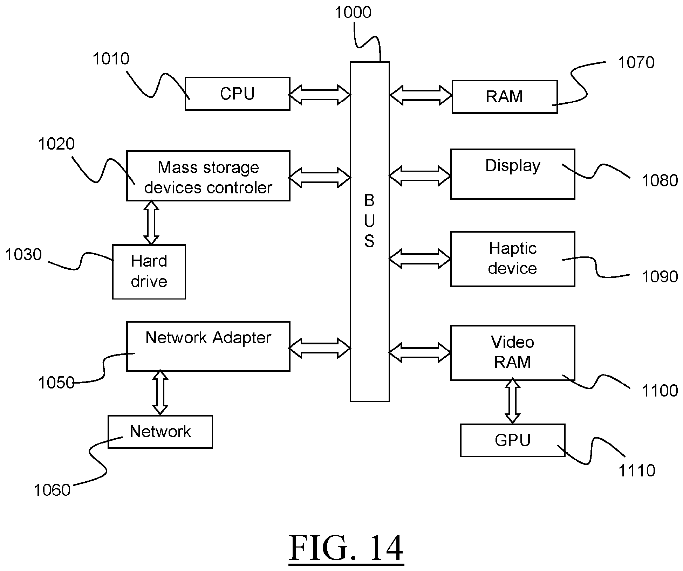

FIG. 14 shows an example of the system, wherein the system is a client computer system, e.g., a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer-implemented method for predicting a hemodynamic vascular behavior of a plurality of arteries of a patient with uncertainty quantification, the method comprising:
- obtaining (S100) one or more pre-trained machine-learning functions, comprising:
∘ obtaining (S101) a pre-trained parameter-signal machine-learning function having been trained for outputting an instance of predetermined hemodynamic signals from an instance of predetermined parameters provided as input, wherein:
• the predetermined hemodynamic signals comprise one or more hemodynamic signals, each of the hemodynamic signals characterizing a blood flow of a respective artery of the plurality of arteries of the patient; and
• the predetermined parameters comprise one or more parameters, each of the parameters characterizing a respective artery of the plurality of arteries of the patient;
∘ obtaining (S102) a pre-trained parameter-noise machine-learning function having been trained for outputting an instance of predetermined characterizations of hemodynamic localization noise from an instance of the predetermined parameters provided as input, wherein:
• the predetermined characterizations of hemodynamic localization noise comprise one or more characterizations of noise, each characterization of noise characterizing a variability of a hemodynamic signal characterizing a blood flow of a respective artery of the plurality of arteries of the patient, the variability being with respect to a location in the artery; and
∘ obtaining (S103) a pre-trained measurement-parameter machine-learning function having been trained for outputting an instance of the predetermined parameters from measurements of the hemodynamic vascular behavior provided as input, wherein:
• the measurements of the hemodynamic vascular behavior comprise instances of hemodynamic signals characterizing the blood flow of a predetermined subset of arteries of the plurality of arteries of the patient;
- obtaining (S110) measurements of the hemodynamical vascular behavior of the patient with a medical measuring device;
- generating (S120) a plurality of noisy measurements of the hemodynamic vascular behavior of the patient, the generating comprising:
∘ providing (S121) as input the obtained measurements of the hemodynamic vascular behavior of the patient to the pre-trained measurement-parameter machine-learning function, thereby obtaining a patient-dependent instance of the predetermined parameters;
∘ providing (S122) as input the obtained patient-dependent instance of the predetermined parameters to the pre-trained parameter-noise machine-learning function, thereby obtaining a patient-dependent instance of the predetermined characterizations of hemodynamic localization noise; and
∘ determining (S123) a patient-dependent noise generator from the patient-dependent instance of the predetermined characterizations of hemodynamic localization noise, the patient-dependent noise generator being able to modify the measurements of the hemodynamic vascular behavior of the patient by adding noise, the noise quantifying possible errors in the measurements of the hemodynamic vascular behavior of the patient with the medical measuring device;
- providing (S130) as input each of the noisy measurements of the hemodynamical vascular behavior of the patient to the pre-trained measurement-parameter machine-learning function, thereby obtaining a plurality of noisy patient-dependent instances of the predetermined parameters; and
- providing (S140) as input each of the obtained plurality of noisy patient-dependent instances of the predetermined parameters to the pre-trained parameter-signal machine-learning function, thereby obtaining a plurality of noisy patient-dependent instances of the predetermined hemodynamic signals.

2. The method of claim 1, further comprising obtaining a noise confidence level of the measurements of the hemodynamical vascular behavior of the patient with the medical measuring device, and wherein the patient-dependent noise generator is further determined from the obtained noise confidence level.

3. The method of any one of claims 1 to 2, wherein:
- the pre-trained parameter-signal machine-learning function comprises a pre-trained parameter-signal neural network model for one or more of the hemodynamic signals of respective arteries of the plurality of arteries of the patient comprised in the predetermined hemodynamic signals, each of said pre-trained parameter-signal neural network models outputting instances of the hemodynamic signal of the respective artery;
- the pre-trained parameter-noise machine-learning function comprises a pre-trained parameter-noise neural network model for one or more characterization of noise characterizing a variability of a hemodynamic signal characterizing a blood flow of a respective artery of the plurality of arteries of the patient comprised in the predetermined characterizations of hemodynamic localization noise, each of said pre-trained parameter-noise neural network models outputting instances of the characterization of noise of the respective artery; and/or
- the pre-trained measurement-parameter machine-learning function comprises a pre-trained measurement-parameter neural network model for one or more parameters characterizing a respective artery of the plurality of arteries of the patient comprised in the predetermined parameters, each of said pre-trained measurement-parameter neural network models outputting instances of the parameter characterizing the respective artery.

4. The method of any one of claims 1 to 3, wherein the obtaining (S101) of the pre-trained parameter-signal machine-learning function comprises:
- obtaining (S151) a parameter-signal dataset comprising entries, each entry comprising an instance of the predetermined parameters and an instance of the predetermined hemodynamic signals; and
- training (S161) a machine-learning function on the parameter-signal dataset, thereby obtaining the pre-trained parameter-signal machine-learning function.

5. The method of claim 4, wherein the obtaining (S151) of the parameter-signal dataset comprises:
- obtaining numerical bounds for each parameter comprised in the predetermined parameters, each of the numerical bounds determining a physiological plausible range of the possible values of the respective parameter in a population;
- generating a list of instances of the predetermined parameters, each instance comprising an instance of each of the parameters comprised in the predetermined parameters, the instance of each of the parameters being generated randomly within the obtained numerical bounds;
- obtaining for each instance of the predetermined parameters comprised in the generated list an instance of the predetermined hemodynamic signals by using a one-dimensional blood flow modeling solver;
- normalizing each of the instances of each of the parameters comprised in the instances of the predetermined parameters comprised in the generated list and each of the instances of each of the hemodynamic signals comprised in the obtained instances of predetermined hemodynamic signals; and optionally wherein
- the one-dimensional blood flow modeling solver is given by the formula: $\left\{\begin{matrix}\frac{\partial A}{\partial t} + \frac{\partial Q}{\partial x} = 0 \\ \frac{\partial Q}{\partial t} + \frac{\partial }{\partial x} \left(α\frac{{Q}^{2}}{A}\right) + \frac{A}{ρ} \frac{\partial P}{\partial x} = - 2 \frac{μ}{ρ} \left({γ}_{v}+2\right) \frac{Q}{A}\end{matrix}\right.$ where *A* is the cross-sectional area, Q is the blood flow-rate, *t* is the time coordinate, *x* is the position within the artery, P is the blood pressure, *α* is a parameter that reflects the radial profile of the longitudinal velocity, *ρ* is the mass density, *µ* is the dynamic viscosity, and ${γ}_{ν} = \frac{\left(α-2\right)}{1 - α}$.

6. The method of claim 5, wherein the obtaining (S102) of the pre-trained parameter-noise machine-learning function comprises:
- obtaining (S152) a parameter-noise dataset, the parameter-noise dataset comprising entries, each entry comprising an instance of the predetermined parameters and an instance of the predetermined characterizations of hemodynamic localization noise; and
- training (S162) a machine-learning function on the parameter-noise dataset, thereby obtaining the pre-trained parameter-noise machine-learning function.

7. The method of claim 6, wherein the obtaining (S152) of the parameter-noise dataset comprises for each given entry of the obtained parameter-signal dataset:
- determining a covariance matrix of each of the instances of the hemodynamic signals associated to the given entry with respect to the localization; and optionally wherein
- the covariance matrix *C^{loc}*(*t, t'*|*p*) is given by the formula: $\begin{matrix}{C}^{loc} \left(t,\left.t′\right|p\right) = {E}_{x} \left[\left(S\left(x,\left.t\right|p\right)-{E}_{x}\left[S\left(x,\left.t\right|p\right)\right]\right)\left(S\left(x,\left.t′\right|p\right)-{E}_{x}\left[S\left(x,\left.t′\right|p\right)\right]\right)\right] \\ = {E}_{x} \left[S\left(x,\left.t\right|p\right)\left(x,\left.t′\right|p\right)\right] - {E}_{x} \left[S\left(x,\left.t\right|p\right)\right] {E}_{x} \left[S\left(x,\left.t′\right|p\right)\right]\end{matrix}$ where S is the instance of the hemodynamic signal characterizing the blood flow, *t* and *t'* are time coordinates, x is the position within the artery, and *p* is the instance of the predetermined parameters comprised in the entry.

8. The method of any one of claims 4 to 7, wherein the obtaining (S103) of the pre-trained measurement-parameter machine-learning function comprises:
- obtaining (S153) a measurement-parameter dataset, the measurement-parameter dataset comprising entries, each entry comprising measurements of the hemodynamic vascular behavior and an instance of the predetermined parameters; and
- training (S163) a machine-learning function on the measurement-parameter dataset, thereby obtaining the pre-trained measurement-parameter machine-learning function.

9. The method of claim 8, wherein the obtaining (S153) of the measurement-parameter dataset comprises for each given entry of the obtained parameter-signal dataset:
- obtaining measurements of the hemodynamic vascular behavior from the instances of the hemodynamic signals characterizing the blood flow of the predetermined subset of arteries comprised in the given entry;
- obtaining noisy measurements of the hemodynamic vascular behavior by adding to the obtained measurements of the hemodynamic vascular a noise;
- solving an inverse problem using Bayes method, thereby obtaining an instance of the predetermined parameters that are associated with the noisy measurements of the hemodynamic vascular behavior; and optionally wherein
- solving the inverse problem comprises solving a non-linear optimization problem by minimizing the negative log-posterior using the formula ${p}^{MAP} = {argmin}_{p ∈ {ε}_{p}} \left(-{\sum }_{i = 1}^{{N}_{O}} \left(\begin{matrix}- \frac{{N}_{T}}{2} log \left(2π\right) - \frac{1}{2} log \left(\left|Σ\right|\right) \\ - \frac{1}{2} {\left({O}_{i}-{M}_{i}^{NN}\left(p\right)\right)}^{T} {Σ}^{- 1} \left({O}_{i}-{M}_{i}^{NN}\left(p\right)\right)\end{matrix}\right) - log \left({π}_{prior}\left(p\right)\right)\right)$ where:
∘ *p^{MAP}* is the instance of the predetermined parameters that are associated with the noisy measurements,
∘ *εₚ* is a space of all the possible instances of the predetermined parameters,
∘ *p* is an instance of the predetermined parameters,
∘ *N_{O}* is a number of hemodynamic signals characterizing the blood flow of a predetermined subset of arteries of the plurality of arteries of the patient,
∘ *N_{T}* is a number of time steps of each of the hemodynamic signals,
∘ *Σ* is a covariance matrix characterizing the noise,
∘ *Oᵢ* is an instance of a i-th hemodynamic signal of the noisy measurements of the hemodynamic vascular behavior,
∘ ${M}_{i}^{NN} \left(p\right)$ is an instance of the i-th hemodynamic signal of the measurements of the hemodynamic vascular behavior obtained from the instance *p* of the predetermined parameters, and
∘ *πₚᵣᵢₒᵣ*(*p*) is the priority density of the parameters.

10. The method of any one of claims 1 to 9, wherein each characterization of noise comprised in the predetermined characterizations of hemodynamic localization noise is a covariance matrix, and the patient-dependent noise generator comprises one or more multivariate normal distributions, each of the multivariate normal distribution determined by an instance of a covariance matrix comprised in the patient-dependent instance of the predetermined characterizations of hemodynamic localization noise.

11. The method of any one of claims 1 to 10, wherein the predetermined hemodynamic signals comprise one or more selected among blood pressure, flow rate, cross-sectional area, velocity of each artery of the plurality of arteries of the patient.

12. The method of any one of claims 1 to 11, wherein the predetermined parameters comprise one or more selected among an anatomical, a physiological, a physical, a model parameters of each artery of the plurality of arteries of the patient, and optionally wherein the predetermined parameters comprise one or more selected among a Young's modulus, a vessel length, a vessel radius, 0D Windkessel model parameters of each artery of the plurality of arteries of the patient.

13. A computer-program comprising instructions which, when the program is executed on a computer, cause the computer to carry out the method of any one of claims 1 to 12.

14. A computer-readable storage medium having recorded thereon the computer-program of claim 13.

15. A system comprising a processor coupled to a memory, the memory having recorded thereon the computer-program of claim 13.

## Amended claims

### Amended claims in accordance with Rule 137(2) EPC.

1. A computer-implemented method for predicting a hemodynamic vascular behavior of a plurality of arteries of a patient with uncertainty quantification, the method comprising:
- obtaining (S100) one or more pre-trained machine-learning functions, comprising:
∘ obtaining (S101) a pre-trained parameter-signal machine-learning function having been trained for outputting an instance of predetermined hemodynamic signals from an instance of predetermined parameters provided as input, wherein:
• the predetermined hemodynamic signals comprise one or more hemodynamic signals, each of the hemodynamic signals characterizing a blood flow of a respective artery of the plurality of arteries of the patient, each of the hemodynamic signals depending on a spatial coordinate, the predetermined hemodynamic signals comprising one or more selected among blood pressure, blood flow rate, cross-sectional area, blood velocity of each artery of the plurality of arteries of the patient and being considered at a specific point of the artery; and
• the predetermined parameters comprise one or more parameters, each of the parameters characterizing a respective artery of the plurality of arteries of the patient, the predetermined parameters comprising one or more selected among a Young's modulus, a vessel length, a vessel radius, and a thickness of a vessel wall of each artery of the plurality of arteries of the patient, and 0D Windkessel model parameters of each terminal artery of the plurality of arteries of the patient;
∘ obtaining (S102) a pre-trained parameter-noise machine-learning function having been trained for outputting an instance of predetermined characterizations of hemodynamic localization noise from an instance of the predetermined parameters provided as input, wherein:
• the predetermined characterizations of hemodynamic localization noise comprise one or more characterizations of noise, each characterization of noise characterizing a variability of a hemodynamic signal characterizing a blood flow of a respective artery of the plurality of arteries of the patient, the variability being with respect to a location in the artery, each characterization of noise characterizing the variability of the hemodynamic signal characterizing the blood flow with respect to the spatial coordinate, each characterization of noise being a covariance matrix; and
∘ obtaining (S103) a pre-trained measurement-parameter machine-learning function having been trained for outputting an instance of the predetermined parameters from measurements of the hemodynamic vascular behavior provided as input, wherein:
• the measurements of the hemodynamic vascular behavior comprise instances of hemodynamic signals characterizing the blood flow of a predetermined subset of arteries of the plurality of arteries of the patient;
- obtaining (S110) measurements of the hemodynamical vascular behavior of the patient with a medical measuring device;
- generating (S120) a plurality of noisy measurements of the hemodynamic vascular behavior of the patient, the generating comprising:
∘ providing (S121) as input the obtained measurements of the hemodynamic vascular behavior of the patient to the pre-trained measurement-parameter machine-learning function, thereby obtaining a patient-dependent instance of the predetermined parameters;
o providing (S122) as input the obtained patient-dependent instance of the predetermined parameters to the pre-trained parameter-noise machine-learning function, thereby obtaining a patient-dependent instance of the predetermined characterizations of hemodynamic localization noise; and
∘ determining (S123) a patient-dependent noise generator from the patient-dependent instance of the predetermined characterizations of hemodynamic localization noise, the patient-dependent noise generator being able to modify the measurements of the hemodynamic vascular behavior of the patient by adding noise, the noise quantifying possible errors in the measurements of the hemodynamic vascular behavior of the patient with the medical measuring device, the patient-dependent noise generator comprising one or more multivariate normal distributions, each of the multivariate normal distribution being determined by an instance of a covariance matrix comprised in the patient-dependent instance of the predetermined characterizations of hemodynamic localization noise;
- providing (S130) as input each of the noisy measurements of the hemodynamical vascular behavior of the patient to the pre-trained measurement-parameter machine-learning function, thereby obtaining a plurality of noisy patient-dependent instances of the predetermined parameters; and
- providing (S140) as input each of the obtained plurality of noisy patient-dependent instances of the predetermined parameters to the pre-trained parameter-signal machine-learning function, thereby obtaining a plurality of noisy patient-dependent instances of the predetermined hemodynamic signals, the obtained plurality of noisy patient-dependent instances of the predetermined hemodynamic signals corresponding to an uncertainty of the prediction, the uncertainty depending on the uncertainty of the obtained measurements.

2. The method of claim 1, further comprising obtaining a noise confidence level of the measurements of the hemodynamical vascular behavior of the patient with the medical measuring device, and wherein the patient-dependent noise generator is further determined from the obtained noise confidence level.

3. The method of any one of claims 1 to 2, wherein:
- the pre-trained parameter-signal machine-learning function comprises a pre-trained parameter-signal neural network model for one or more of the hemodynamic signals of respective arteries of the plurality of arteries of the patient comprised in the predetermined hemodynamic signals, each of said pre-trained parameter-signal neural network models outputting instances of the hemodynamic signal of the respective artery;
- the pre-trained parameter-noise machine-learning function comprises a pre-trained parameter-noise neural network model for one or more characterization of noise characterizing a variability of a hemodynamic signal characterizing a blood flow of a respective artery of the plurality of arteries of the patient comprised in the predetermined characterizations of hemodynamic localization noise, each of said pre-trained parameter-noise neural network models outputting instances of the characterization of noise of the respective artery; and/or
- the pre-trained measurement-parameter machine-learning function comprises a pre-trained measurement-parameter neural network model for one or more parameters characterizing a respective artery of the plurality of arteries of the patient comprised in the predetermined parameters, each of said pre-trained measurement-parameter neural network models outputting instances of the parameter characterizing the respective artery.

4. The method of any one of claims 1 to 3, wherein the obtaining (S101) of the pre-trained parameter-signal machine-learning function comprises:
- obtaining (S151) a parameter-signal dataset comprising entries, each entry comprising an instance of the predetermined parameters and an instance of the predetermined hemodynamic signals; and
- training (5161) a machine-learning function on the parameter-signal dataset, thereby obtaining the pre-trained parameter-signal machine-learning function.

5. The method of claim 4, wherein the obtaining (S151) of the parameter-signal dataset comprises:
- obtaining numerical bounds for each parameter comprised in the predetermined parameters, each of the numerical bounds determining a physiological plausible range of the possible values of the respective parameter in a population;
- generating a list of instances of the predetermined parameters, each instance comprising an instance of each of the parameters comprised in the predetermined parameters, the instance of each of the parameters being generated randomly within the obtained numerical bounds;
- obtaining for each instance of the predetermined parameters comprised in the generated list an instance of the predetermined hemodynamic signals by using a one-dimensional blood flow modeling solver;
- normalizing each of the instances of each of the parameters comprised in the instances of the predetermined parameters comprised in the generated list and each of the instances of each of the hemodynamic signals comprised in the obtained instances of predetermined hemodynamic signals; and optionally wherein
- the one-dimensional blood flow modeling solver is given by the formula: $\left\{\begin{matrix}\frac{\partial A}{\partial t} + \frac{\partial Q}{\partial x} = 0 \\ \frac{\partial Q}{\partial t} + \frac{\partial }{\partial x} \left(α\frac{{Q}^{2}}{A}\right) + \frac{A}{ρ} \frac{\partial P}{\partial x} = - 2 \frac{μ}{ρ} \left({γ}_{v}+2\right) \frac{Q}{A}\end{matrix}\right.$ where *A* is the cross-sectional area, Q is the blood flow-rate, *t* is the time coordinate, x is the position within the artery, P is the blood pressure, *α* is a parameter that reflects the radial profile of the longitudinal velocity, *ρ* is the mass density, *µ* is the dynamic viscosity, and ${γ}_{ν} = \frac{\left(α-2\right)}{1 - α}$.

6. The method of claim 5, wherein the obtaining (S102) of the pre-trained parameter-noise machine-learning function comprises:
- obtaining (S152) a parameter-noise dataset, the parameter-noise dataset comprising entries, each entry comprising an instance of the predetermined parameters and an instance of the predetermined characterizations of hemodynamic localization noise; and
- training (S162) a machine-learning function on the parameter-noise dataset, thereby obtaining the pre-trained parameter-noise machine-learning function.

7. The method of claim 6, wherein the obtaining (S152) of the parameter-noise dataset comprises for each given entry of the obtained parameter-signal dataset:
- determining a covariance matrix of each of the instances of the hemodynamic signals associated to the given entry with respect to the localization; and optionally wherein
- the covariance matrix *C^{loc}*(*t, t*'|*p*) is given by the formula:$\begin{matrix}{C}^{loc} \left(t,t′\left|p\right.\right) = {E}_{x} \left[\left(S\left(x,t\left|p\right.\right)-{E}_{x}\left[S\left(x,t\left|p\right.\right)\right]\right)\left(S\left(x,t′\left|p\right.\right)-{E}_{x}\left[S\left(x,t′\left|p\right.\right)\right]\right)\right] \\ = {E}_{x} \left[S\left(x,t\left|p\right.\right)S\left(x,t′\left|p\right.\right)\right] - {E}_{x} \left[S\left(x,t\left|p\right.\right)\right] {E}_{x} \left[S\left(x,t′\left|p\right.\right)\right]\end{matrix}$ where S is the instance of the hemodynamic signal characterizing the blood flow, *t* and *t'* are time coordinates, x is the position within the artery, and *p* is the instance of the predetermined parameters comprised in the entry.

8. The method of any one of claims 4 to 7, wherein the obtaining (S103) of the pre-trained measurement-parameter machine-learning function comprises:
- obtaining (S153) a measurement-parameter dataset, the measurement-parameter dataset comprising entries, each entry comprising measurements of the hemodynamic vascular behavior and an instance of the predetermined parameters; and
- training (S163) a machine-learning function on the measurement-parameter dataset, thereby obtaining the pre-trained measurement-parameter machine-learning function.

9. The method of claim 8, wherein the obtaining (S153) of the measurement-parameter dataset comprises for each given entry of the obtained parameter-signal dataset:
- obtaining measurements of the hemodynamic vascular behavior from the instances of the hemodynamic signals characterizing the blood flow of the predetermined subset of arteries comprised in the given entry;
- obtaining noisy measurements of the hemodynamic vascular behavior by adding to the obtained measurements of the hemodynamic vascular a noise;
- solving an inverse problem using Bayes method, thereby obtaining an instance of the predetermined parameters that are associated with the noisy measurements of the hemodynamic vascular behavior; and optionally wherein
- solving the inverse problem comprises solving a non-linear optimization problem by minimizing the negative log-posterior using the formula ${p}^{MAP} = {argmin}_{p ∈ {E}_{p}} \left(-{\sum }_{i = 1}^{{N}_{O}} \left(\begin{matrix}- \frac{{N}_{T}}{2} log \left(2π\right) - \frac{1}{2} log \left(\left|Σ\right|\right) \\ - \frac{1}{2} {\left({O}_{i}-{M}_{i}^{NN}\left(p\right)\right)}^{T} {Σ}^{- 1} \left({O}_{i}-{M}_{i}^{NN}\left(p\right)\right)\end{matrix}\right) - log \left({π}_{prior}\left(p\right)\right)\right)$ where:
∘ *p^{MAP}* is the instance of the predetermined parameters that are associated with the noisy measurements,
∘ *εₚ* is a space of all the possible instances of the predetermined parameters,
∘ *p* is an instance of the predetermined parameters,
∘ *N_{O}* is a number of hemodynamic signals characterizing the blood flow of a predetermined subset of arteries of the plurality of arteries of the patient,
∘ *N_{T}* is a number of time steps of each of the hemodynamic signals,
∘ *Σ* is a covariance matrix characterizing the noise,
∘ *Oᵢ* is an instance of a i-th hemodynamic signal of the noisy measurements of the hemodynamic vascular behavior,
∘ ${M}_{i}^{NN} \left(p\right)$ is an instance of the i-th hemodynamic signal of the measurements of the hemodynamic vascular behavior obtained from the instance p of the predetermined parameters, and
∘ *πₚᵣᵢₒᵣ*(*p*) is the priority density of the parameters.

10. The method of any one of claims 1 to 9, wherein the predetermined parameters comprise one or more selected among an anatomical, a physiological, a physical, a model parameters of each artery of the plurality of arteries of the patient.

11. A computer-program comprising instructions which, when the program is executed on a computer, cause the computer to carry out the method of any one of claims 1 to 10.

12. A computer-readable storage medium having recorded thereon the computer-program of claim 11.

13. A system comprising a processor coupled to a memory, the memory having recorded thereon the computer-program of claim 11.
